# EUROPEAN PATENT SPECIFICATION

(11) **EP 1 320 362 B1**
(45) Date of publication and mention of the grant of the patent: **31.08.2011**
(21) Application number: 01928684.8
(22) Date of filing: 20.04.2001
(51) Int. Cl.: A61K 31/216, A61P 3/06, A61K 9/14

(54) **STABILISED FIBRATE MICROPARTICLES**
STABILISIERTE FIBRAT-MIKROPARTIKEL
MICROPARTICULES DE FIBRATE STABILISEES

(30) Priority: 20.09.2000 US 234186 P; 20.10.2000 US 241761 P
(43) Date of publication of application: 25.06.2003
(73) Proprietor: Jagotec AG, 4132 Muttenz (CH)
(72) Inventor: GUIVARC'H, Pol-Henri, Montreal, Quebec H3L 3C1 (CA); PACE, Gary, Winchester, MA 01890 (US); SNOW, Robert, A., West Chester, PA 19380 (US)
(74) Representative: Bevan, Emma
(86) International application number: PCT/US2001/012826
(87) International publication number: WO 2002/024193

(56) References cited:
- WO-A-00/30616
- WO-A-01/80828
- WO-A-98/35666
- GENEST J JR ET AL: "Effect of micronized fenofibrate on plasma lipoprotein levels and hemostatic parameters of hypertriglyceridemic patients with low levels of high-density lipoprotein cholesterol in the fed and fasted state", JOURNAL OF CARDIOVASCULAR PHARMACOLOGY, RAVEN PRESS, NEW YORK, NY LNKD- DOI:10.1097/00005344-200001000-00022, vol. 35, no. 1, 1 January 2000 (2000-01-01), pages 164-172, XP009120945, ISSN: 0160-2446

## Description

The present invention relates to the use of a composition comprising fenofibrate microparticles and a phospholipid surface active substance to reduce variance in the bioavailability of fenofibrate in a mammal in a fasting state versus a mammal in a fed state, in the manufacture of a medicament for the treatment of dislipidemia or dislipoproteinemia by oral administration of said medicament to the mammal such that the bioavailability of fenofibrate in a fasting mammal is increased by less than 25 % compared with the bioavailability of fenofibrate in a fed mammal and/or such that the bioavailability of fenofibrate is increased by a factor of 1.14 in a fasted mammal versus a fed mammal.

It has long been known that the bioavailability of many hydrophobic drugs can be improved if the drugs are administered with food, i.e., the drugs' uptake into the blood or other part of the body exhibit a food effect. A patient is often instructed to take the drug at meal times or with food. Various explanations of the food effect have been advanced including delayed gastric emptying to allow more drug to dissolve before reaching the small intestine thereby producing longer residence times at specific absorption sites in the small intestine; direct interaction and solubilization of drug by food, especially by hydrophobic food components such as fats and lipids; food-related increases in hepatic blood flow to cause a decrease in first-pass metabolism; and increased gastrointestinal secretions that can improve drug solubility.

Dosage forms or quantities of compositions containing a fibrate such as fenofibrate have been marketed and prescribed for the treatment of dislipidemia and dislipoproteinemia. Dislipidemia and dislipoproteinemia are herein defined to include the group selected from hypercholesterolemia, abnormal and elevated levels of cholesterol, abnormal and elevated levels of LDL cholesterol, abnormal and elevated levels of total cholesterol, abnormal and elevated levels of plasma cholesterol, abnormal and elevated levels of triglycerides, hypertrigylceridaemia, abnormal levels of lipoproteins, abnormal and elevated levels of low density lipoproteins (LDLs), abnormal and elevated levels of very low density lipoproteins, abnormal and elevated levels of very low intermediate density lipoproteins, abnormal levels of high density lipoprotein, hyperlipidemia, hyperchylomicronemia, abnormal levels of chylomicrons, related disorders, and combinations thereof such as those described in The JLIB Lipid Handbook for Clinical Practice, Blood Lipids and Coronary Heart Disease, Second Edition, A.M. Gotto et al, International lipid Information Bureau, New York, NY, 2000.

Elevation of serum cholesterol, triglyercides, or both is characteristic of hyperlipidemias. Differentiation of specific abnormalities usually requires identification of specific lipoprotein fractions in the serum of a patient. Lipoproteins transport serum lipids and can be identified by their density and electrophoretic mobility. Chylomicrons are among the largest and least dense of the lipoproteins. Others, in order of increasing density and decreasing size include very low density lipoproteins (VLDL or pre-beta), intermediate low density lipoproteins (ILDL or broad-beta), low density lipoproteins (LDL or beta), and high density lipoproteins (HDL or alpha). Triglycerides are transported primarily by chylomicrons and very low density lipoproteins. Cholesterol is transported primarily by low density lipoproteins. Hyperlipidemia types include type I, type IIa, type IIb, type III, type IV, and type V. These types can be characterized according to the levels relative to normal of lipids (cholesterol and triglycerides) and lipoproteins described above. Hyperlipidemia types are listed in Table 1 below, wherein "N" refers to normal levels of the substance in the left column, "+" refers to slightly elevated levels, "+ +" refers to elevated levels, "-" refers to slightly decreased levels, and "- -" refers to decreased levels, all relative to normal. The data in the table are derived from Drug Facts and Comparisons, 52nd Edition (1998) page 1066: Treatment of a patient presenting one of more of the symptoms listed in Table 1 by the method of treatment and composition of the dosage forms described herein will lead to a lowering in elevated levels of lipids and lipoproteins in the patient.

| Table 1. Hyperlipidemia types as a function of relative Lipid and Lipoprotein levels. | | | | | | |
|---|---|---|---|---|---|---|
| Hyperlipidemia type | I | II a | II b | III | IV | V |
| Lipids | | | | | | |
| Cholesterol | N+ | ++ | ++ | N++ | N+ | N++ |
| Triglycerides | ++ | N | ++ | N++ | ++ | ++ |

| Lipoproteins | | | | | | |
|---|---|---|---|---|---|---|
| Chylomicrons | ++ | N | N | N | N | ++ |
| VLDL (pre-beta) | N+ | N- | ++ | N+ | ++ | ++ |
| ILDL (broad-beta) | | | | ++ | | |
| LDL (beta) | -- | ++ | ++ | ++ | N- | -- |
| HDL(alpha) | -- | N | N | N | N- | -- |

Fibrates used as lipid regulating agents in the treatment of lipid disorders include fenofibrate (brand name TRICOR), bezafibrate (brand name BEZALIP), clofibrate (brand name ATROMID-S), gemfibrozil (brand nmae LOPID), and ciprofibrate. Preferred fibrates are water-insoluble or poorly water soluble compounds, and preferably solids, either amorphous or crystalline.

Fibrates can act as prodrugs and be metabolized in vivo to provide species that are active species in the treatment of hyperlipidemia. The major metabolite of fenofibrate found in plasma is fenofibric acid, a fibrate active species which has an elimination half-life of approximately twenty hours. Fenofibric acid lowers plasma triglycerides by potentially inhibiting triglyceride synthesis leading to a reduciton of VLDL released into the circulation. Fenofibric acid also stimulates the catabolism of triglyceride-rich lipoprotein (VLDL). Measurement of the detected amount of fenofibric acid in the blood of a patient can reflect the efficacy of fenofibrate uptake.

Fenofibrate also reduces serum une acid levels in hyperuricemic and normal individuals by increasing the urinary excretion of uric acid. The compositions described herein are also useful in the reduction of uric acid leve

Fenofibrate or 2-[4-(4-chlorobenzoyl)phenoxy]-2-methyl-propanoic acid 1-methylethyl ester is an example of a poorly water soluble compound. It is a benzophenone containing a para-chlorophenyl group and a para-isopropyloxycarbonylisopropoxyphenyl group, both of which are substantially hydrophobic groups. Fenofibrate exhibits a melting point reported to be in the range of 79 to 82 °C (Physician's Desk Reference, 1999 Edition, page 477), which is above that of the symmetrically unsubstituted benzophenone with a reported melting point range of 48 to 51 °C but below that of the symmetrically substituted 4,4'-dichlorobenzophenone with a reported range of 144 to 146 °C (Aldrich Chemical Co. catalog, 1999).

Fenofibrate acts as a potent lipid modulator agent offering unique and significant clinical advantages over existing products in the fibrate class of drugs. Fenofibrate produces substantial reductions in plasma triglycetide levels in hypertriglyceridemic patients and in plasma cholesterol and LDL-cholesterol in hypercholesterolemic and mixed dyslipidemic patients.

Fenofibrate is a prodrug that is absorbed and then hydrolyzed by tissue and plasma esterases to fenofibric acid, its active metabolize or active species. Fenofibric acid, responsible for the pharmacological activity, has a plasma half-life of about 20 hours. Fenofibrate is a poorly water soluble drug and is practically insoluble in water. It is normally poorly and variably absorbed, and currently is prescribed to be taken with food.

There have been a number of improvement in dosage forms of fenofibrate in an effort to increase bioavailability of the drug and hence its efficacy. However, there is still a need for a dosage formulation that can substantially reduce or overcome the differential between the bioavailability of the drug in patients who are fasted versus the bioavailability of the drug in patients who are fed.

Fenofibrate was first available in a pharmaceutical dosage form (Lipidil®) consisting of a hard gelatin capsule containing fenofibrate, lactose, pregelatinized starch and magnesium stearate. After oral administration, during a meal, about 60% of the dose of this conventional form is effectively absorbed and found in the blood as fenofibric acid (Weil et al., The metabolism and disposition of 14C-fenofibrate in human volunteers, Drug. Metabol. Dispos. Biol. Fate. Chem., 18 (1990) 115-120).

Historically, in order to improve the intestinal absorption, another pharmaceutical dosage form was introduced (Lipidil Micro®). European Patent Application 330,532 and U.S. patent 4,895,726 disclose a fenofibrate composition in which fenofibrate powder is co-micronized with a solid wetting agent. Sodium lauryl sulfate is described as the wetting agent of choice. The co-micronized powder so obtained is mixed with capsule filling excipients such as lactose, starch, cross-linked polyvinyl pyrrolidone (PVP), and magnesium stearate. A study comparing this formulation (Lipidil Micro®) to the conventional form (Lipidil®) had showed statistically significant increase in bioavailability with the former. A formulation of fenofibrate that refers to this patent is currently available in the United States under the name TRICOR MICRONIZED®.

European Patent Application 724,877 describes fenofibrate powder co-micronized with a wetting agent in association with a vitamin E component (tocopherol and/or its organic acid ester) for treating or preventing disorders associated with lipoprotein oxidation.

U.S. patent 4,800,079 describes a medicinal composition in the form of granules with controlled release of fenofibrate. Each granule includes an inert core, a layer based on fenofibrate and a protective layer. Fenofibrate is present in the form of crystalline microparticles of dimensions not greater than 30 µm.

U.S. patent 4,961,890 describes a process for preparing a controlled release formulation containing fenofibrate in an intermediate layer in the form of crystalline microparticles (less than 30 µm in diameter) within a multilayer layer inert matrix.

European Patent Application 757,911 describes a fenofibrate pharmaceutical dosage form in which fenofibrate is in solution in diethylene glycol monoethyl ether (EMDG) which is a non-ionic surfactant.

European Patent Application No. EP0793958A2 discloses a process for producing a fenofibrate solid dosage form utilizing fenofibrate, a surface active agent and polyvinyl pyrrolidone in which the fenofibrate particles are mixed with a polyvinyl pyrrolidone solution. The thus obtained mixture is granulated with an aqueous solution of one or more surface active agents, and the granulate thus produced is dried.

European Patent Application 904,781 describes a process for making granules of a solid dispersion of a disintegrant in molten fenofibrate by blending a solid dispersing agent into molten fenofibrate, cooling and solidifying the bulk mixture in a tray, and then milling the solid through a screen to produce granules. Disintegrants include polymers such as starch, croscarmellose sodium, sodium starch glycolate, and crospovidone. Such disintegrants are slow to swell and dissolve in aqueous media. Furthermore, when crosslinked as in the case of crospovidone, a polymeric disintegrant will not be uniformly dissolved in molten drug but rather at best will form micro-domains in molten fenofibrate. In addition, polymeric materials can exhibit phase separation phenomena when distributed in a substance with which there is not complete compatibility. This was shown, in part, by Sheu, M. T. et al., "Characterization and dissolution of fenofibrate solid dispersion systems", Int. J. Pharm. (1994), 103(2), 137-46 using differential scanning calorimetry measurements that found fenofibrate to be incompatible with poly(vinyl pyrrolidone). Thus, preparation of a bulk mixture in the melt followed by solidification and grinding can lead to non-uniform distributions and compositions in granules. This can adversely effect the bioavailability of the active component.

U.S. patent 5,700,471 discloses a process for the micronization of compounds having low solubility in water by exposing such compounds briefly to a temperature above their respective melting points, dispersing them with turbulence in an aqueous or organic phase, and subsequently cooling the phase to form a fine particle dispersion. However, it is specified (column 2, lines 1-9) that certain substances and specifically fenofibrate are not amenable to processing entirely without organic solvents because their aqueous dispersions agglomerate and cannot be metered. Thus, in example 2 of U.S. patent 5,700,471, fenofibrate is not directly dispersed in water but rather is first dissolved in a four-fold excess of a water-miscible organic solvent (isopropanol) which must be removed in a subsequent step. Organic solvents can pose flammability risks, exposure dangers to process operators, potential environmental problems, and added expense related to their storage, ultimate removal from a formulation, and disposal. Thus it is desirable to overcome the use of organic solvents where possible.

U.S. patent 4,880,634 describes a method of production of an excipient system containing a pharmacologically active substance for peroral administration comprised of lipid nano-pellets in an aqueous, colloidal suspension. The method comprises forming a melt of a mixture of at least one surfactant, a pharmacologically active substance, and at least one lipid, dispersing the molten mixture within an aqueous solution at a temperature above the melting point of the lipid to form lipid nano-pellets, and cooling the suspension below the melting point of the lipid. In the process, a pharmacologically effective substance is dissolved in the lipid or mixture of lipids during the preparation of the lipid nano-pellets. Animal and plant phospholipids such as lecithin and their hydrogenated forms may be employed in the process although the use of chloroform is taught in examples citing phospholipon 100H. The pharmacologically effective substance can be added to the melted lipid in molten form or dissolved or dispersed in the molten lipid.

U.S. Patent 4,895,726 discloses a gelatin capsule dosage form of fenofibrate containing a co-micronized mixture of particles of fenofibrate and a solid surfactant. The dosage form exhibits improved dissolution rate and bioavailability of fenofibrate over that of micronized fenofibrate alone or that of micronized fenofibrate subsequently mixed with solid surfactant. However, the surfactant must be a solid so it can be micronized, and the micronized surfactant in the form of particles is not uniformly juxtaposed or coated on the surface of the fenofibrate particles.

U. S. Patent 6,180,138 discloses a process for the preparation of solid formulations of a lipid-regulating agent including fenofibrate having enhanced dissolution and absorption characteristics, in which a micronized mixture of the lipid-regulating agent, and optionally one or more excipients, is suspended in a surfactant solution, dried by spray drying, optionally granulated, and optionally converted into a finished capsule or tablet dosage form.

WO 97/13503 discloses a method of synthesizing nanoparticle composites by combining an agent and a matrix to form a composite mixture in an organic solvent or solvent/water, and then spray drying to remove the solvent.

WO 00/40220 discloses a method for making microparticles by dissolving a water insoluble drug in an organic solvent and a water soluble polymer in an organic solvent, mixing the two solutions, and spray drying to obtain microparticles. To increase bioavailability of the drug, the particles are mixed with an oil.

U.S. Patent 5,545,628 discloses a melted and cooled pharmaceutical composition in a hard gelatin capsule for treating hyperlipidemia and/or hypercholesterolemia. The composition contains fenofibrate, one or more polyglycolyzed glycerides, and optionally other polyalkylene glycol polymers that are added to adjust HLB value, melting point, and stabiliity. The composition provides an increased bioavailability of fenofibrate with respect to previously marketed forms of fenofibrate (i.e., non co-micronized Lypantyl 200 RTM., and co-micronized Lypantyl 200 M.RTM.). Commercially available formulations of fenofibrate such as TRICOR Micronized exhibit a food effect, for example, the amount of fenofibrate taken up and metabolized to the active fibrate species, fenofibric acid, depends on the amount and kind of food taken proximal (within about +/- one or two hours before or after) the time of taking the fenofibrate oral dosage form (e.g., capsule or tablet).

Ben-Armor solubilized fenofibrate in nonaqueous dimethyl isosorbide with a miscible wetting agent to improve its bioavailability. Colloidal silicon oxide was added to increase the viscosity, and the liquid so obtained was placed in hard gelatin capsules and sealed. In vivo studies with this formulation indicated no statistically significant difference in bioavailability between the liquid formulation and a conventional form when the product was given with food.

U.S. Patents 5,645,856 and 6,096,338 disclose a composition and method of improving the in vivo bioavailability of a hydrophobic drug from a pharmaceutical composition comprising the drug dispersed or dissolved in a digestible oil containing a hydrophilic surfactant which substantially inhibits the in vivo lipolysis of the digestible oil, wherein there is added to the composition a lipophilic surfactant capable of reducing the inhibitory effect of the hydrophilic surfactant. They also disclose a carrier system for a hydrophobic drug which comprises a digestible oil and a pharmaceutically acceptable surfactant for dispersing the oil in vivo upon administration of the carrier system, the surfactant comprising a hydrophilic surfactant component which substantially inhibits the in vivo lipolysis of the digestible oil, and a lipophilic surfactant component capable of reducing the inhibitory effect of the hydrophilic surfactant component.

U.S. Patents 5,776,495 and 6,027,747 disclose a solid dispersion with enhanced bioavailability of a surface active agent and at least one therapeutic agent in a hydrophilic carrier having enhanced solubility in an aqueous medium. The dispersion is prepared by dissolving the therapeutic agent in a volatile organic solvent containing a very hydrophilic polymer and without strong heat or vacuum evaporating the solvent to dryness to form a coprecipitate of therapeutic agent and hydrophilic polymer.

U.S. Patent 5,827,536 discloses soluble fenofibrate pharmaceutical dosage formulations exhibiting improved bioavailability after oral administration. However, the formulations contain fenofibrate as a solution in a solubilizing agent consisting of diethylene glycol monoethyl ether.

U.S. Patent 6,042,847 discloses a three-phase pharmaceutical form exhibiting constant and controlled release of an amorphous active ingredient stabilized with polymers for a single daily peroral application. The first phase consists of a core containing an amorphous active ingredient, polyvinylpyrrolidone and a cellulose ether as carriers and as inhibitors of its crystallization, and a surfactant that improves the solubility of the active ingredient and promotes the absorption of the amorphous active ingredient from gastrointestinal tract. The second phase contains a cellulose ether and a mixture of mono-, di- and triglycerides as sustained release agents. The third phase is a poorly soluble or gastro-resistant polymeric film coating.

U.S. Patent 6,068,854 discloses a constant release tablet consisting of a matrix of gelatin in which is dispersed as an emulsion, dispersion or colloid a lipophilic and/or poorly water soluble pharmaceutical substance with a particle size below 200 micrometers.

WO 2000037057 discloses a solution formulation comprising a lipid-regulating agent dissolved in at least one propylene glycol fatty acid ester as the primary solvent medium for the agent, optionally together with one or more emulsifiers including phospholipids.

WO 2000016749 discloses a formulation comprising a solution of a lipid-regulating agent dissolved in at least one propylene glycol fatty acid ester as the primary solvent medium for the agent. One or more emulsifiers may be added to the formulation.

WO 98/31361 discloses a pharmaceutical composition of fenofibrate with high biological availability and method for preparing same. The invention concerns a fenofibrate composition with instant release comprising and inert water-soluble support coated with at least a film containing an active fenofibrate principle in micronized form with a size less than 20 micrometers, a hydrophilic polymer and optionally a surfactant, and optionally one or several external phases or films.

U.S. Patent 5,880,148 discloses a combination of fenofibrate and a vitamin E substance where the fenofibrate is micronized with a solid surfactant.

U.S. Patent 6,074,670 discloses an immediate-release fenofibrate composition comprising an inert hydrosoluble carrier covered with a layer containing fenofibrate in a micronized form having a size less than 20 micrometers, a hydrophilic polymer and, optionally, a surfactant. In an example cited, a suspension of micronized fenofibrate and sodium lauryl sulfate is suspended in a solution of sodium lauryl sulfate and polyvinylpyrrolidone, sprayed onto 100 to 400 micrometers size lactose particles suspended in a fluidized air bed granulator, and the granulate is placed in capsules or transformed into tablets by mixing with cross-linked PVP, microcrystalline cellulose, colloidal silica, and sodium stearyl fumarate. The composition showed enhanced bioavailability of fenofibrate. However, increased dissolution rates of a formulation of fenofibrate do not translate directly or linearly to increase uptake of the drug, and show that an in vitro experimental result can not necessarily predict the results of an in vivo experiment.

It is generally accepted that water insoluble or poorly water soluble drugs can be made more bioavailable when presented in the form of small particles. In many cases, it is known that small particles must be stabilized against particle size growth and agglomeration by the addition of one or more surface active agents at some point in the preparation of the particles, especially in a size reduction process that employs the input of mechanical energy such as homogenization, microfluidization, milling, such as media milling, precipitation such as from a liquified gas, ball milling and the like. Because they are biocompatible and well tolerated in vivo, preferred surface active agents or particle stabilizers are phospholipids, and preferred small particles of fenofibrate are stabilized by phospholipid particle stabilizers that are also referred to herein as phospholipid surface active substances or species. A phospholipid surface active substance can be a single phospholipid compound or a mixture of phospholipid compounds, a natural phospholipid isolated for example from plants such as soy or animal sourses such as hen egg, or a synthetic phospholipid. Phospholipids that are isolated from plants or animals can be purified into different grades of phospholipids including grades sold for use in food and grades sold for use in pharmaceuticals. For example, Lipoid E 80 may contain phosphatidyl choline, phosphatidyl ethanolamine, lysophosphatidyl choline, lysophosphatidyl ethanolamine, sphingomyelin, and trace quantities of triglycerides, cholesterol, free fatty acids, d,1-alpha-tocopherol, and water.

Microparticles of water insoluble or poorly soluble substances are small particles having diameters of from nanometers to micrometers and refer to solid particles of irregular, non-spherical or spherical shapes. When the insoluble and poorly soluble substances are therapeutically and diagnostically useful substances, formulations containing them as microparticles or small particles provide some specific advantages over unformulated non-micronized drug particles. These advantages include improved oral bioavailability of drugs that are poorly absorbed from the GI tract, development of injectable formulations that are currently available only in oral dosage form, less toxic injectable formulations that are currently prepared with organic solvents, sustained release of intramuscular injectable drugs that are currently administered through daily injection or constant infusion, preparation of inhaled and ophthalmic formulations of drugs that otherwise could not be formulated for nasal or ocular use, as well as other advantages.

Current technology for delivering insoluble drugs as described in U.S. Pat. Nos. 5,091,188; 5,091,187 and 4,725,442 focuses on (a) either coating small drug particles with surface active substances that are natural or synthetic phospholipids or (b) dissolving the drug in a suitable lipophilic carrier and forming an emulsion stabilized with surface active substances that are natural or semisynthetic phospholipids.

U.S. Patent 5,145,684 discloses methods for preparation and dispersions of particles consisting of crystalline drug substance having a surface modifier or surface active substance adsorbed to maintain an effective average particle size of less than about 400 nm. However, the method requires a milling step that can result in impurities being added to the formulation from fractured milling media.

U.S. Patents 5,470,583 and 5,336,507 disclose methods for preparation of nanoparticles using a charged phospholipid as a cloud point modifier.

U.S. Patent 5,302,401 discloses nanoparticles having a surface modifier adsorbed on the surface of the particles and a cryoprotectant associated therewith. The cryoprotectant is present in an amount sufficient to allow the nanoparticles to be lyophilized.

International Patent Application WO 99/39700 describes the preparation of submicron nanoparticles from a pharmacologically active principle and a composite material consisting of at least one lipidic substance and at least one amphiphilic substance using high pressure homogenization to form a microemulsion of the composite material at a temperature higher than the melting temperature of at least one of the materials forming the composite and in the presence of one or more aqueous surfactants as surface active substances and then cooling the microemulsion to form a dispersion of solid particles.

U.S. Patent 5,785,976 discloses a heated aqueous emulsification and cooling process for the preparation of solid lipid particles. In that process a solid lipid or bioactive agent or a mixture of solid lipids or bioactive agents is melted and stabilizers, i.e., surface active substances, are added either to the lipid or bioactive agent and to the aqueous phase or to the aqueous phase only. The aqueous phase is heated to the temperature of the melt before mixing and may contain stabilizers, isotonicity agents, buffering substances, cryoprotectants and/or preservatives. The molten lipid compounds and the bioactive agents can be emulsified in the aqueous phase by high-pressure homogenization. The homogenized dispersion is then allowed to cool until solid particles are formed by recrystallization of the dispersed agents. Drugs or other bioactive substances to be incorporated into the particles may be melted together with the lipids or may be dissolved, solubilized or dispersed in the lipid melt before an emulsification by homogenization step.

U.S. Patent 5,922,355 discloses a method for preparing submicron size microparticles by particle size reduction methods in which a solid material is reduced in size over a period of time while continuously below the melting point of the material or by precipitation while the particles are stabilized with phospholipids as surface active substances in combination with other surface modifiers to control growth of particle size and enhance storage stability. The use of one or more surface modifiers in addition to a phospholipid provides volume weighted mean particle size values that are much smaller than what can be achieved using phospholipid alone without the use of an additional surface active substance (surfactant) with the same energy input while providing compositions resistant to particle size growth on storage. The phospholipid and the surfactant are both present at the time of particle size reduction.

WO 00/30616 discloses a rapidly dispersing solid dry dosage form comprised of a water insoluble compound existing as a nanometer or micrometer particulate solid which is surface stabilized by the presence of at least one phospholipid, the particulate solid being dispersed throughout a bulking matrix. When the dosage form is introduced into an aqueous environment, the bulking matrix is substantially completely dissolved within less than 2 minutes thereby releasing the water insoluble particulate solid in an unaggregated and/or unagglomerated state. The matrix is composed of a water insoluble substance or therapeutically useful water insoluble or poorly water soluble compound, a phospholipid and optionally also at least one non-ionic, anionic, cationic, or amphiphatic surfactant, together with a matrix or bulking agent and if needed a release agent. The volume weighted mean particle size of the water insoluble particle is 5 micrometers or less.

In one aspect while it is advantageous in very many cases to use particulate pharmaceutical formulations wherein particle sizes are stabilized by combinations of phospholipids and surface modifiers according to U.S. Patent 5,922,355, it is sometimes desirable to produce pharmaceutical formulations or pre-formulations which are stabilized by biocompatible phospholipids without the use of additional surface active substances. This can be desirable, for example, when there is a subsequent need to modify the composition of a particle-containing formulation in a step following the formation of the particles such as by the addition of one or more additional ingredients that are not compatible with additional surface modifiers shown to be beneficial in U.S. Patent 5,922,355.

In one aspect it is therefore desirable to produce drug particles stabilized by one or more phospholipids in the absence of additional surface modifiers but which exhibit enhanced stability toward particle growth and which maintain sub-micron and micron size particles on subsequent storage as suspension or solid dosage form.

In another aspect, particle size reduction methods such as those disclosed in U.S. 5,922,355 in which particles of a material are reduced in size in the presence of phospholipid and another surface active substance while the material is maintained in the solid phase require processing for a certain length of time to achieve a desired particle size. The time is directly related to the number of homogenization volume passes or turnovers performed on a volume of a suspension of particles in a size reduction process. It is desirable to further reduce that length of time by providing an improved process that can decrease the overall number of turnovers to achieve a desired particle size.

While these disclosures provide compositions and methods to enhance the bioavailabilty of fibrates such as fenofibrate from various dosage forms, none sufficiently address the need to substantially reduce or eliminate the difference between the amount of the drug taken up in patients who are fasting versus the otherwise enhanced uptake of the drug in patients who are fed or take food with or proximal to the taking of a dosage form of a fibrate.

D. Fleischer, Cheng Li, Yuji Zhou, Li-Heng Pao and Aziz Karim in "Drug, Meal and Formulation Interactions Influencing Drug Absorption After Oral Administration," Clin. Pharmacokinet, (1999), Mar:36 (3), 233-264 review information regarding oral drug/meal interaction effects on GI drug absorption.

GENEST J JR ET AL: "Effect of micronized fenofibrate on plasma lipoprotein levels and hemostatic parameters of hypertriglyceridemic patients with low levels of high-density lipoprotein cholesterol in the fed and fasted state", JOURNAL OF CARDIOVASCULAR PHARMACOLOGY, vol. 35, no. 1, 1 January 2000 (2000-01-01), pages 164-172, discloses the use of compositions comprising fenofibrate microparticles (Tricor or Lipidil) for the treatment of dislipidemia and hypertriglyceridemia and shows that the compound is effective in the fed and fasted state.

It is thus an object of this disclosure to provide to a mammal such as a human patient an oral pharmaceutical dosage form of a fibrate for use in the treatment of dislipidemia and dislipoproteinemia and related disorders of whereby such a dosage form substantially reduces or substantially eliminates the difference in the amount of the drag or active fibrate species taken up in the patient when in a fasting state versus the amount taken up using the same dosage level in the same patient when in a fed state.

### BRIEF SUMMARY OF THE INVENTION

The present invention provides the use of a composition comprising fenofibrate microparticles and a phospholipid surface active substance to reduce variance in the bioavailability of fenofibrate in a mammal in a fasting state versus a mammal in a fed state, in the manufacture of a medicament for the treatment of dislipidemia or dislipoproteinemia by oral administration of said medicament to the mammal such that the bioavailability of fenofibrate in a fasting mammal is increased by less than 25 % compared with the bioavailability of fenofibrate in a fed mammal and/or such that the bioavailability of fenofibrate is increased by a factor of 1.14 in a fasted mammal versus a fed mammal.

The pharmaceutically effective composition for use in the invention comprises small particles of fenofibrate stabilized by a phospholipid stabilizing agent which when dried in the presence of a sugar and optionally also in the presence of a carbohydrate-derived alcohol can be formulated as a capsule or tablet or powder or granular dosage form for oral administration to a patient in need of treatment by fenofibrate. The dosage form provides dosage levels of fenofibrate active species into the blood of a patient in a fasted or fed state wherein the amount of drug or active species that the patient receives in the fasted state differs by less than 25%, preferably by less than 20%, more preferably by less than 15%, even more preferably by less than 10%, and most preferably by less than 5% from the amount of drug or active species that the patient receives in the fed state.

In a conical study using capsule dosage forms and monitoring the pharmacokinetic comparison of a single dose of a phospholipid-stabilized fenofibrate formulation for use in this invention versus a comicronized fenofibrate (Lipanthyl 67M) in healthy volunteers under fed and fasted conditions, distinct advantages are seen. For example, under fasted conditions, it was unexpectedly found that the formulation for use in this invention provided a statistically significant increased relative bioavailability of approximately 1.5 times that of the comicronized formulation as evidenced by an 84% higher mean maximum concentration (Cₘₐₓ) of the drug and approximately 50% higher mean AUC's. This significant difference between the two formulations disappeared under fed conditions.

When the bioavailability of the comicronized formulation under fed versus fasted conditions was compared, the Cₘₐₓ significantly increased by 211% and the mean AUC's significantly increased by over 70%. In addition, the mean terminal half-life appeared to be shortened.

In contrast and unexpectedly, when the bioavailability of the formulation for use in this invention under fed versus fasted conditions was compared, the Cₘₐₓ significantly increased by only 61% and the mean AUC's were increased by only 13%. The relative bioavailability was approximately 1.14 when comparing fasted versus fed conditions using the formulation for use in this invention. No significant variation in mean terminal half-life was observed.

The phospholipid-stabilized fibrate particle formulation for use in this invention provides a pharmacokinetic profile in which the effect of ingestion of food on the uptake of the drug is substantially reduced (even up to the point of elimination of the effect of the ingestion of food) over that observed with the commercially available comicronized formulation. In a preferred aspect, the phospholipid-stabilized fenofibrate particle formulation for use in this invention provides a pharmacokinetic profile in which the effect of ingestion of food on the uptake of the drug is substantially reduced over that observed with the commercially available comicronized formulation.

The small particles or microparticles of solid fibrate for use in this invention are prepared in the presence of a phospholipid surface active agent as a particle stabilizer. Preferred methods of preparation include the methods of Haynes disclosed in US Patents 5,091,187 and 5,091,188 and by improved processes described herein as well as the methods in US Serial No. 60/198,579 and US Serial No. 60/203,366. Other useful methods of preparation include the methods of Parikh et al disclosed in US Patent 5,922,355, PCT/US99/13755 and potentially other milling methods such as ball milling, media milling and the like for example such as disclosed in US 4,727,077, 4,006,025, and 4,294,916 if these methods are applied using a phospholipid or a mixture of phospholipids as a particle stabilizer,

Small particles or microparticles of fenofibrate for use in this invention are conveniently prepared by an energy input process, and especially by a microfluidization process to provide the small particles in the form of an aqueous suspension. The microfluidization process is a wet or aqueous, one- or two-stage size reduction process that is done in the presence of a liquefied or vesicular surface active agent (e.g., one or more pharmaceutically acceptable phospholipids such as a single phospholipid or a mixture of phospholipids such as soy-derived phospholipid, egg phospholipid, and especially Lipoid E80 - a purified egg phospholipid, natural phospholipids, synthetic phospholipids, purified natural phospholipids, fractions of natural phospholipids, charged anionic or cationic phospholipids, and mixtures thereof), and optionally in the presence of pharmaceutically acceptable additives or excipients such as sucrose, sorbitol, mannitol and the like, other surface active agents, and preferably in an aqueous buffer such as an aqueous sodium phosphate buffer. When the microfluidization is done in two stages or processing steps wherein the first stage is run at a temperature above the melting point of the drug and the second stage is run below the melting point of the drug, we refer to such a process as a hot melt microfluidization process. Water is then subsequently removed from the suspension for example by a lyophilization (i.e., a freeze-drying step) to form a dried lyophilized and substantially dry powder comprising the solid particles of fenofibrate,. The water can also be removed by other means such as by spray drying.

Small particles of fenofibrate for use in this invention stabilized by phospholipid can be prepared as a suspension by a process comprising the steps of (a) mixing at high shear an admixture of a poorly water soluble drug and one or more than one surface active substance in an aqueous carrier in the absence of an organic solvent within a first temperature range at or above the melting point of the poorly water soluble drug to form a heated suspension containing the drug, then (b) homogenizing said heated suspension in a first pressure range and within said first temperature range to form a heated homogenate containing the drug, then (c) cooling said heated homogenate to a second temperature range below the melting temperature of the poorly water soluble drug to form a transiently stable cooled homogenate containing the drug, then (d) applying a particle stabilizing energetic process to said cooled homogenate within a second temperature range below the melting point of the drug and in a second pressure range to form a cooled dispersion of stabilized small particles containing the drug, and then (e) optionally drying the cooled dispersion to form dried small particles containing the poorly water soluble drug.

In a typical procedure, a premix of fenofibrate, phospholipid Lipoid E80 (dispensed frozen but liquefied or vesiclized at processing temperatures), sorbitol, and sucrose in 10 millimolar aqueous phosphate buffer at pH 8 is microfluidized above the melting temperature of fenofibrate for about 3 to 10 volume passes, cooled, and further microfluidized for another 10 volume passes to form a suspension of microparticles of fenofibrate stabilized by phospholipid in aqueous sorbitol/sucrose/phosphate buffer.

Particularly important to the preparation of the composition for use in this invention is the use of two homogenization steps separated by a cooling step. The first homogenization step is done on a heated suspension having the poorly water soluble drug in a molten phase in the presence of one or more than one surface active substance to provide a heated homogenate containing the drug. The heated homogenate is usually in the form of a microemulsion comprising small molten particles or droplets of drug stabilized by one or more than one surface active substance. The heated homogenate containing the drug is then cooled to provide a transiently stable cooled homogenate containing the drug. The transiently stable cooled homogenate comprises small particles of drug in which the drug is in a solid phase which may be amorphous, crystalline, or a combination of both. The small particles of the cooled homogenate are stabilized by the surface active substance or substances but the particles are transiently stable with respect to particle size growth and eventual precipitation of solid drug from the aqueous carrier.

The second homogenization step is done on the cooled homogenate after a cooling step to produce a cooled dispersion of small particles containing the drug and having greater stability to particle growth and precipitation than the cooled homogenate. The second homogenization step is a stabilizing energetic process. It provides small particles that are more stable than the transiently stable particles of the cooled homogenate prepared in the first homogenization step and prevents relatively large crystals and/or agglomerates of the poorly water soluble drug from forming. The second homogenization step thereby facilitates the formation of stabilized small particles of the poorly water soluble drug. It also provides overall rapid formation of desired small particles containing the poorly water soluble drug. Optionally, the small particles can be isolated by a drying process, for example by lyophilization or by spray drying. Thus, the process can provide dried small particles containing a poorly water soluble drug. In the absence of the second homogenization step, very large amounts of the poorly water soluble drug can precipitate from the transiently stable aqueous cooled homogenate or can form a sediment by precipitation from the aqueous carrier.

By "dried" we mean having a water or moisture content greater than zero per cent and below 5% by weight, preferably below 4% by weight, more preferably below 3% by weight, and even more preferably below 2% by weight, and most preferably below 1% by weight. In preferred embodiments, the amount of water is between 0.1% and 3%, more preferably between 0.1% and 2%, and most preferably between 0.1% and 1% by weight. By "anhydrous" we mean have zero water content.

As described herein, we have unexpectedly found that small particles containing the poorly water soluble drug fenofibrate can be prepared by a process comprising the steps of (a) mixing at high shear an admixture of fenofibrate and one or more than one surface active substance in an aqueous carrier in the absence of an organic solvent within a first temperature range above the melting point of fenofibrate to form a heated suspension containing fenofibrate, then (b) homogenizing said heated suspension in a first pressure range and within said first temperature range to form a heated homogenate containing fenofibrate, then (c) cooling said heated homogenate to a second temperature range below the melting temperature of fenofibrate to form a transiently stable cooled homogenate containing fenofibrate, then (d) applying a particle stabilizing energetic process to said cooled homogenate within a second temperature range and in a second pressure range to form a cooled dispersion of stabilized small particles containing fenofibrate, and then (e) optionally drying the cooled dispersion to form dried small particles containing fenofibrate. Particularly important to this aspect of the disclosure is the use of two homogenization steps separated by a cooling step and the use of a phospholipid as a surface active substance. The first homogenization step is done on a heated suspension in the presence of a phospholipid as a surface active substance, in the absence of an organic solvent, and wherein fenofibrate is molten to provide a homogenized microemulsion containing fenofibrate. The second homogenization step is done on a transiently stable cooled homogenate in the presence of the phospholipid and wherein the fenofibrate is a solid to provide a homogenized dispersion of small particles containing fenofibrate. In the absence of the second homogenization step, relatively large crystals of fenofibrate readily form from the transiently stable cooled homogenate. In the absence of a heated first homogenization step on the molten drug, homogenization of solid fenofibrate to provide a suspension of small particles of fenofibrate takes a prolonged or much longer time in the same homogenization apparatus under substantially the same homogenization conditions of pressure and temperature relative to the time taken in the second homogenization step described herein.

In a preferred aspect, the average particle size of the cooled dispersion generally increases during the second homogenization step rather than decreases, and thus it is not generally a size reduction step.

It is an advantage of this invention that a composition of a pharmaceutical dosage form of fenofibrate is provided that substantially reduces the difference between the amount of the drug taken up in patients who are fasting versus the amount of the drug in patients who are fed.

It is another advantage of this invention that a once-a-day pharmaceutically effective dosage form of fenofibrate is provided that can be administered orally to a patient in need of treatment by the drug without regard to the amount of food a patient has ingested prior to or following administration of the dosage form. These and other advantages will be readily apparent from the description of the invention.

### BRIEF DESCRIPTION OF THE DRAWINGS

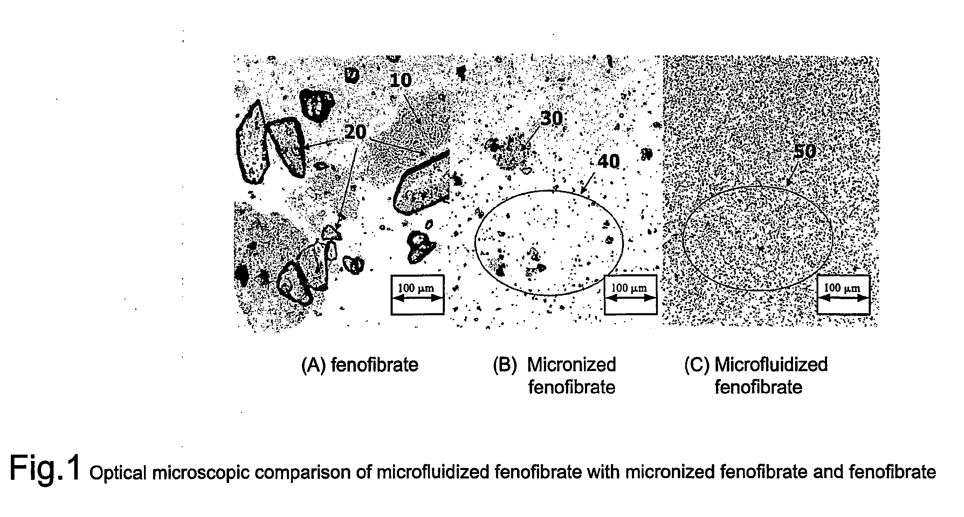

Figure 1 is an optical microscopic comparison of microfluidized fenofibrate with micronized fenofibrate and fenofibrate compositions prepared in the presence of starch.

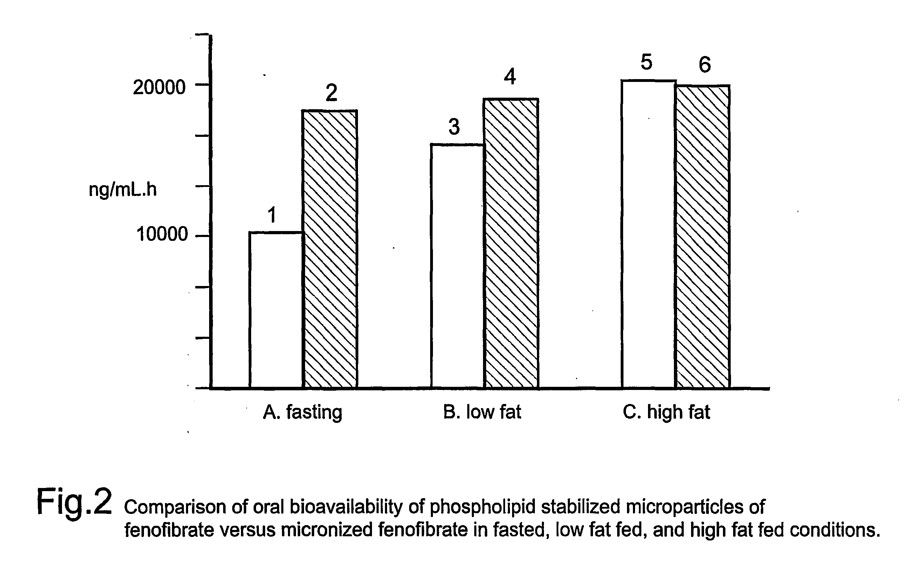

Figure 2 is a comparison of the oral bioavailability of microparticles of fenofibrate prepared by microfluidization in the presence of a phospholipid stabilizing agent versus the oral bioavailability of micronized fenofibrate under fasting, low fat fed, and high fat fed conditions.

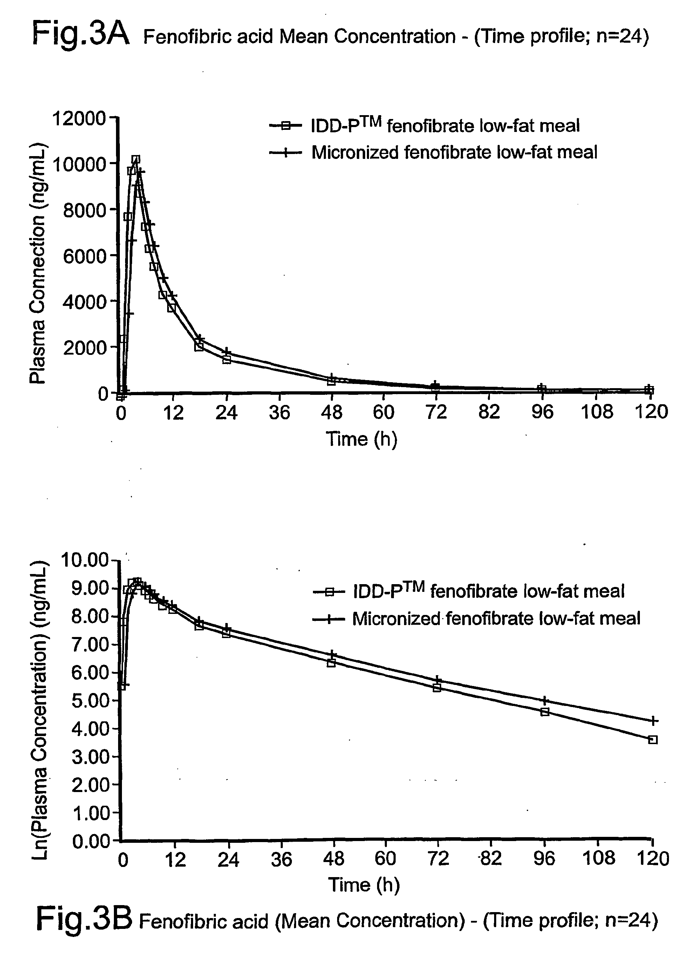

Figure 3A is a graph of fenofibric acid mean plasma concentration (in ng/ml) versus time (in hours) found after oral administration of a 160 mg fenofibrate-containing tablet prepared as described herein compared to that of a commercially available 200 mg Tricor® capsule each taken proximal to ingestion of a low fat meal (n=24).
Figure 3B is a graph of fenofibric acid Ln mean plasma concentration (in ng/ml) versus time (in hours) found after oral administration of a 160 mg fenofibrate-containing tablet prepared as described herein compared to that of a commercially available 200 mg Tricor® capsule each taken proximal to ingestion of a low fat meal (n=24).

### DETAILED DESCRIPTION OF THE INVENTION

Described herein is a method of treating dislipidemia and dislipoproteinemia in a mammal which method comprises administering to said mammal a therapeutically effective oral dosage form comprising microparticles of a solid fibrate that are stabilized by a phospholipid surface active substance wherein said dosage form provides into the blood of said patient in a fasted state a therapeutically effective amount of said fibrate that is at least 90% of the AUC amount of said fibrate provided by said dosage form into the blood of said patient in a fed state. AUC refers to area under the curve.

Also described herein is a method of treating dislipidemia and dislipoproteinemia in a human patient which method comprises administering to said patient a therapeutically effective oral dosage form comprising microparticles of a solid fenofibrate that are stabilized by a phospholipid surface active substance wherein said dosage form provides into the blood of said patient in a fasted state a therapeutically effective amount of fenofibrate active species that is at least 90% of the AUC amount of said fenofibrate active species provided by said dosage form into the blood of said patient in a fed state.

Also described herein is an orally administered pharmaceutical composition comprising microparticles of solid fibrate that are stabilized by a phospholipid surface active substance, wherein said microparticles are prepared in the presence of said phospholipid surface active substance, and wherein a therapeutically effective amount of said composition provides a quantity of fibrate active species to a fasted human patient in need of treatment by said fibrate that is greater than 90% of the quantity of said fibrate active species provided by said amount to said patient when fed a high fat meal.

Also described herein is an orally administered pharmaceutical composition comprising microparticles of solid fenofibrate that are stabilized by a phospholipid surface active substance, wherein said microparticles are prepared in the presence of said phospholipid surface active substance, and wherein a therapeutically effective amount of said composition provides a quantity of fenofibrate active species to a fasted human patient in need of treatment by said fenofibrate that is greater than 90% of the quantity of said fenofibrate active species provided by said amount to said patient when fed a high fat meal.

Also described herein is a pharmaceutically effective composition comprising small particles of a fibrate stabilized by a phospholipid stabilizing agent which when dried in the presence of a sugar and optionally also in the presence of a carbohydrate-derived alcohol can be formulated as a capsule or tablet or powder or granular dosage form for oral administration to patients in need of treatment by said fibrate. The dosage form provides dosage levels of drug or fibrate active species into the blood of a patient in a fasted or fed state wherein the amount of drug or active species that the patient receives in the fasted state differs by less than 25%, preferably by less than 20%, more preferably by less than 15%, even more preferably by less than 10%, and most preferably by less than 5% from the amount of drug or active species that the patient receives in the fed state.

Also described herein is a pharmaceutically effective composition comprising small particles of fenofibrate stabilized by a phospholipid stabilizing agent which when dried in the presence of a sugar and optionally also in the presence of a carbohydrate-derived alcohol can be formulated as a capsule or tablet or powder or granular dosage form for oral administration to a patient in need of treatment by fenofibrate. The dosage form provides dosage levels of fenofibrate active species into the blood of a patient in a fasted or fed state wherein the amount of drug or active species that the patient receives in the fasted state differs by less than 25%, preferably by less than 20%, more preferably by less than 15%, even more preferably by less than 10%, and most preferably by less than 5% from the amount of drug or active species that the patient receives in the fed state.

Also described herein is an orally administered pharmaceutical composition comprising microparticles of solid fenofibrate that are stabilized by a phospholipid surface active substance, wherein said microparticles are prepared in the presence of said phospholipid surface active substance, and wherein a therapeutically effective amount of said composition provides a quantity of fenofibrate active species to a fasted human patient in need of treatment by fenofibrate that is greater than 80% of the quantity of fenofibrate active species provided by said amount to said patient when fed a high fat meal comprising at least 1000 calories 50 % of which are from fat.

Also described herein is an orally administered pharmaceutical composition comprising microparticles of solid fenofibrate that are stabilized by a phospholipid surface active substance, wherein said microparticles are prepared in the presence of said phospholipid surface active substance and one or more excipients, and wherein a therapeutically effective amount of said composition provides a quantity of fenofibrate active species to a fasted human patient in need of treatment by fenofibrate that is greater than 80% of the quantity of fenofibrate active species provided by said amount to said patient when fed a high fat meal comprising at least 1000 calories 50 % of which are from fat.

As used herein, a fasted patient is defined as a patient who has not eaten any food, i.e., has fasted for at least 10 hours before the administration of a dosage form of a drug such as fenofibrate and who does not eat any food and continues to fast for at least 4 hours after the administration of the dosage form. The dosage form is administered with 180 ml of water during the fasting period, and water can be allowed ad libitum after 2 hours.

As used herein, a fed patient is defined as a patient who has fasted for at least 10 hours overnight and then has consumed an entire test meal within 30 minutes of first ingestion. The dosage form is administered with 180 ml of water within 5 minutes after completion of the meal. No food is then allowed for at least 4 hours post-dose. Water can be allowed ad libitum after 2 hours. A high fat test meal provides approximately 1000 calories to the patient of which approximately 50% of the caloric content is derived from fat content of the meal. A representative high fat high caloric test meal comprises 2 eggs fried in butter, 2 strips of bacon, 2 slices of toast with butter, 4 ounces of hash brown potatoes, and 8 ounces of whole milk to provide 150 protein calories, 250 carbohydrate calories, and 500 to 600 fat calories. High fat meals can be used in clinical bioequivalence and bioavailability studies of fenofibrate. High fat meals promote increased absorption and uptake of fenofibrate.

The absence or elimination of a food effect can be concluded when the 90% confidence intervals for the ratio of the geometric means based on log-transformed data in clinical studies of fed and fasted treatments fall within 80% to 125% for AUC (area under the concentration time curve) and 70% to 143% for Cₘₐₓ (peak concentration). The presence of a food effect can be concluded when the 90% confidence intervals for the ratio of the geometric means based on log-transformed data in clinical studies of fed and fasted treatments fall outside 80% to 125% for AUC and outside 70% to 143% for Cₘₐₓ.

As used herein, "small particle" refers to a particle or a distribution of particles having a diameter or an average diameter, respectively, of from nanometers to micrometers. Small particles are microparticles, as used herein, and also refer to solid particles of irregular, non-spherical or spherical shapes.

By "transiently stable" we mean that the small particles of the cooled homogenate remain as small particles in a dispersion of the aqueous carrier at the size finally produced in the first homogenization step for a relatively short period of time and not indefinitely. The period of time that a cooled homogenate remains transiently stable can vary from up to about one second to up to about 48 hours, and preferably from up to about 15 minutes to up to about 24 hours, and most preferably from up to about 6 hours to up to about 24 hours although the period of time can vary with many factors. For example as commonly seen in recrystallization of a crystalline substance from an organic solvent, the growth and precipitation of crystals can be induced or enhanced by the presence of seed crystals, by stirring of a cooled supersaturated solution of drug, and by scratching the internal surface of a vessel containing supersaturated dissolved drug below the level of the liquid thereby creating nucleation sites for crystallization. Such crystal growth is not desirable in the present invention. The transiently stable particles can grow slightly in size (i.e., in average diameter) over the relatively short period of time by as much as 1000% of their original size or more from that size produced in the heated homogenization step, but preferably will remain at the size at which they were produced in the first homogenization step up to a size about 100% larger in diameter, and more preferably up to a size about 50% larger in diameter. After the relatively short period of time, the particles will continue to become larger such as by Ostwald ripening and crystallization. After the relatively short period of time, drug may also crystallize in the form of large particles from the suspension. The particles of the heated homogenate may also irreversibly agglomerate after the relatively short period of time. Additionally, after the relatively short period of time, the components of the formulation may phase separate from the aqueous carrier and optionally precipitate and separate into components that contain largely drug and largely surface active substance. This is not desired.

Water insoluble and poorly water soluble compounds are those having poor solubility in water at or below normal physiological temperatures, that is <5 mg/ml at physiological pH (6.5-7.4). Preferably their water solubility is <1 mg/ml, and more preferably <0.1 mg/ml. It is desirable that the drug be stable in water as a dispersion. Otherwise or in addition a dried form such as a lyophilized or spray-dried solid form may be desirable for example for use in formation of drug delivery compositions including capsules, tablets, powder granules, and formulations with additional excipients and drugs.

Examples of some preferred water-insoluble drugs that are also suitable for preparation into small particles and dosage forms as described herein include immunosuppressive and immunoactive agents, antiviral and antifungal agents, antineoplastic agents, analgesic and anti-inflammatory agents, antibiotics, anti-epileptics, anesthetics, hypnotics, sedatives, antipsychotic agents, neuroleptic agents, antidepressants, anxiolytics, anticonvulsant agents antagonists, neuron blocking agents, anticholinergic and cholinomimetic agents, antimuscarinic and muscarinic agents, antiadrenergic and antarrhythmics, antihypertensive agents, antineoplastic agents, hormones, and nutrients. A detailed description of these and other suitable drugs may be found in Remington's Phamaceutical Sciences, 18th edition, 1990, Mack Publishing Co. Philadelphia, Pennsylvania.

Drugs that are poorly soluble in water can have pharmaceutical efficacy in a number of therapeutic and diagnostic imaging areas. Non-limiting classes of compounds and agents from which poorly water soluble drugs that melt without decomposition and are useful as described herein can be selected include anesthetic agents, ace inhibiting agents, antithrombotic agents, anti-allergic agents, antibacterial agents, antibiotic agents, anticoagulant agents, anticancer agents, antidiabetic agents, antihypertension agents, antifungal agents, antihypotensive agents, antiinflammatory agents, antimicotic agents, antimigraine agents, antiparkinson agents, antirheumatic agents, antithrombins; antiviral agents, beta blockering agents, bronchospamolytic agents, calcium antagonists, cardiovascular agents, cardiac glycosidic agents, carotenoids, cephalosporins, contraceptive agents, cytostatic agents, diuretic agents, enkephalins, fibrinolytic agents, growth hormones, immunosurpressants, insulins, interferons, lactation inhibiting agents, lipid-lowering agents, lymphokines, neurologic agents, prostacyclins, prostaglandins, psycho-pharmaceutical agents, protease inhibitors, magnetic resonance diagnostic imaging agents, reproductive control hormones, sedative agents, sex hormones, somatostatins, steroid hormonal agents, vaccines, vasodilating agents, and vitamins.

Preferred drugs suitable for processing into small particles as described herein melt without decomposition in admixtures, suspensions, dispersions, and homogenates, preferably in a temperature range from about physiological temperature 37 °C to about 275 °C, and more preferably in a temperature range from just above physiological temperature, about 40 °C, to about 230 °C. In one aspect, preferred suitable drugs melt without decomposition in the range from physiological temperature at about 37 °C to the boiling point of water at atmospheric pressure, i.e., up to about 100 °C but not including 100 °C unless a reflux condenser is present to control the loss of liquid. In this case, the aqueous carrier can be maintained at the first temperature range generally without the need of pressurization to maintain the aqueous carrier as a liquid during the heated homogenization process. In another aspect of this invention, preferred suitable drugs melt without decomposition in the range from the boiling point of the aqueous carrier under ambient pressure, i.e., from about 100 °C up to 275 °C. In this case, the aqueous carrier can be maintained at the first temperature range generally by using a pressurized apparatus to maintain the aqueous carrier as a liquid during the heated homogenization process. Of course, if desired, a pressurized apparatus can be used in the range below the boiling point of the aqueous carrier such as in the region of from 50 °C to about 100 °C, and the aqueous carrier will also be maintained as a liquid.

Non-limiting examples of representative poorly soluble drugs suitable for use in the hot melt process that is described herein for the preparation of microparticles of fenofibrate stabilized with one or more (i.e., a mixture of) phospholipid stabilizing agents and that melt without decomposition in admixtures, suspensions, dispersions, and homogenates as described herein at temperatures at or below 275 °C can be selected from the group consisting albendazole (m.p. 208-210 °C), albendazole sulfoxide, alfaxalone (m.p. 172-174 °C), acetyl digoxin, acyclovir analogs melting at or below 275 °C, alprostadil, aminofostin, anipamil, antithrombin III, atenolol (m.p. 146-148 °C), azidothymidine, beclobrate (m.p. 200-204 °C), beclomethasone (m.p. 117-120 °C), belomycin, benzocaine (m.p. 88-90 °C) and derivatives beta carotene (m.p. 183 °C), beta endorphin, beta interferon, bezafibrate (m.p. 186 °C), binovum, biperiden (m.p. 112-116 °C), bromazepam (mp. 237-238 °C), bromocryptine, bucindolol, buflomedil (m.p. 192-193 °C), bupivacaine (m.p. 107-108 °C), busulfan (m.p. 114-118 °C), cadralazine_{.} (m.p. 160-162 °C), camptothesin (m.p. 264-267 and 275 °C), canthaxanthin (m.p. 217 °C), captopril (m.p. 103-104 °C), carbamazepine (m.p. 190-193 °C), carboprost, cefalexin, cefalotin, cefamandole (m.p. 190 °C), cefazedone, cefluoroxime, cefmenoxime, cefoperazone (m.p. 169-171 °C), cefotaxime, cefoxitin (m.p. 149-150 °C), cefsulodin (m.p. 175 °C), ceftizoxime, chlorambucil (m.p. 64-66 °C), chromoglycinic acid, ciclonicate (m.p.127-128 °C), ciglitazone, clonidine (m.p. 130 °C), cortexolone, corticosterone (m.p. 180-182 °C), cortisol (m.p. 212-220 °C), cortisone (m.p. 220-224 °C), cyclophosphamide (m.p. 41-45 °C), cyclosporin A (m.p. 148-151 °C) and other cyclosporins, cytarabine (m.p. 212-213 °C), desocryptin, desogestrel (m.p. 109-110 °C), dexamethasone esters such as the acetate (m.p. 238-240 °C), dezocine, diazepam (m.p. 125-126 °C), diclofenac, dideoxyadenosine (m.p. 160-163 °C), dideoxyinosine, digitoxin (m.p. 256-257 °C), digoxin, dihydroergotamine (m.p. 239 °C), dihydroergotoxin, diltiazem (m.p. 207-212 °C), dopamine antagonists, doxorubicin (m.p. 229-231 °C), econazole (m.p. 87 °C), endralazine (m.p. 185-188 °C), enkephalin, enalapril (m.p. 143-145 °C), epoprostenol, estradiol (m.p. 173-179 °C), estramustine (m.p. 104-105 °C), etofibrate (m.p. 100 °C), etoposide (m.p. 236-251 °C), factor ix, factor viii, felbamate (m.p. 151-152 °C), fenbendazole (m.p. 233 °C), fenofibrate (m.p. 79-82 °C), flunarizin (m.p. 252 °C), flurbiprofen (m.p. 110-111 °C), 5-fluorouracil (m.p. 282-283 °C), flurazepam (m.p. 77-82 °C), fosfomycin (m.p. ∼94 °C), fosmidomycin, furosemide (m.p. 206 °C), gallopamil, gamma interferon, gentamicin (m.p. 102-108 °C), gepefrine (m.p. 155-158 °C), gliclazide (m.p. 180-182 °C), glipizide (m.p. 208-209 °C), griseofulvin (m.p. 220 °C), haptoglobulin, hepatitis B vaccine, hydralazine (m.p. 172-173 °C), hydrochlorothiazide (m.p. 273-275 °C), hydrocortisone (m.p. 212-220 °C), ibuprofen (m.p. 75-77 °C), ibuproxam (m.p. 119-121 °C), indinavir, indomethacin (m.p. 155 °C), iodinated aromatic x-ray contrast agents melting below 275 °C such as iodamide (m.p. 255-257 °C), ipratropium bromide (m.p. 230-232 °C), ketoconazole (m.p. 146 °C), ketoprofen (m.p. 94 °C), ketotifen (m.p. 152-153 °C), ketotifen fumarate (m.p. 192 °C), K-strophanthin (m.p. ∼175 °C), labetalol, lactobacillus vaccine, lidocaine (m.p. 68-69 °C), lidoflazin (m.p. 159-161 °C), lisuride (m.p. 186 °C), lisuride hydrogen maleate (m.p. 200 °C), lorazepam (m.p. 166-168 °C), lovastatin, mefenamic acid (m.p. 230-231 °C), melphalan (m.p. 182-183 °C), memantin, mesulergin, metergoline (m.p. 146-149°C), methotrexate (m.p. 185-204 °C), methyl digoxin (m.p. 227-231 °C), methylprednisolone (m.p. 228-237 °C), metronidazole (m.p. 158-160 °C), metisoprenol, metipranolol (m.p. 105-107 °C), metkephamide, metolazone (m.p. 253-259 °C), metoprolol, metoprolol tartrate, miconazole (m.p. 135 °C), miconazole nitrate (m.p. 170 and 185 °C), minoxidil (m.p. 248 °C), misonidazol, molsidomin, nadolol (m.p. 124-136 °C), nafiverine (m.p. 220-221 °C), nafazatrom, naproxen (m.p. 155 °C), natural insulins, nesapidil, nicardipine (m.p. 168-170 °C), nicorandil (m.p. 92-93 °C), nifedipine (m.p. 172-174 °C), niludipin, nimodipine, nitrazepam (m.p. 224-226 °C), nitrendipine, nitrocamptothesin, 9-nitrocamptothesin, oxazepam (m.p. 205-206 °C), oxprenolol (m.p. 78-80 °C), oxytetracycline (m.p. 181-182 °C), penicillins such as penicillin G benethamine (m.p. 147-147 °C), penecillin O (m.p. 79-81 °C), phenylbutazone (m.p. 105 °C), picotamide, pindolol (m.p. 171-173 °C), piposulfan (m.p. 175-177 °C), piretanide (m.p. 225-227 °C), piribedil (m.p. 98 °C), piroxicam (m.p. 198-200 °C), pirprofen (m.p. 98-100 °C), plasminogenic activator, prednisolone (m.p. 240-241 °C), prednisone (m.p. 233-235 °C), pregnenolone (m.p. 193 °C), procarbacin, procaterol, progesterone (m.p. 121 °C), proinsulin, propafenone, propanolol, propentofyllin, propofol, propranolol (m.p. 96 °C), rifapentin, simvastatin, semi-synthetic insulins, sobrerol (m.p. 130 °C), somastotine and its derivatives, somatropin, stilamine, sulfinalol whose hydrochloride melts at 175 °C, sulfinpyrazone (m.p. 136-137 °C), suloctidil (m.p. 62-63 °C), suprofen (m.p. 124 °C), sulproston, synthetic insulins, talinolol (m.p. 142-144 °C), taxol, taxotere, testosterone (m.p. 155 °C), testosterone propionate (m.p. 118-122 °C), testosterone undecanoate, tetracane HI (m.p. ∼150 °C), tiaramide (HCl m.p. 159-161 °C), tolmetin (m.p. 155-157 °C), tranilast (m.p. 211-213 °C), triquilar, tromantadine (HCl m.p. 157-158 °C), urokinase, valium (m.p. 125-126 °C), verapamil (m.p. 243-246 °C), vidarabine, vidarabine phosphate sodium salt, vinblastine (m.p. 211-216 °C), vinburin, vincamine (m.p. 232-233 °C), vincristine (m.p. 218-220 °C), vindesine (m.p. 230-232 °C), vinpocetine (m.p. 147-153 °C), vitamin A (m.p. 62-64 °C), vitamin E succinate (m.p. 76-78 °C), and x-ray contrast agents. Drugs can be neutral species or basic or acidic as well as salts such as exist in the presence of an aqueous buffer,

While compositions of microfluidized fenofibrate stabilized with a phospholipid surface active agent and formulated as described herein provide substantial reduction to elimination of the food effect that is observed with other formulations of fenofibrate, the hot melt method of production of such microparticles has application to other drugs, especially water insoluble or poorly water soluble drugs and to other surface active substances.

Examples of some suitable surface active substances that are useful in the hot melt microfluidization process include: (a) natural surfactants such as casein, gelatin, tragacanth, waxes, enteric resins, paraffin, acacia, gelatin, cholesterol esters and triglycerides, (b) nonionic surfactants such as polyoxyethylene fatty alcohol ethers, sorbitan fatty acid esters, polyoxyethylene fatty acid esters, sorbitan esters, glycerol monostearate, polyethylene glycols, cetyl alcohol, cetostearyl alcohol, stearyl alcohol, poloxamers, polaxamines, methylcellulose, hydroxycellulose, hydroxy propylcellulose, hydroxy propylmethylcellulose, noncrystalline cellulose, polyvinyl alcohol, polyvinylpyrrolidone, and synthetic phospholipids, (c) anionic surfactants such as potassium laurate, triethanolamine stearate, sodium lauryl sulfate, alkyl polyoxyethylene sulfates, sodium alginate, dioctyl sodium sulfosuccinate, negatively charged phospholipids (phosphatidyl glycerol, phosphatidyl inosite, phosphatidylserine, phosphatidic acid and their salts), and negatively charged glyceryl esters, sodium carboxymethylcellulose, and calcium carboxymethylcellulose, (d) cationic surfactants such as quaternary ammonium compounds, benzalkonium chloride, cetyltrimethylammonium bromide, chitosans and lauryldimethylbenzylammonium chloride, (e) colloidal clays such as bentonite and veegum. A detailed description of these surfactants may be found in Remington's Pharmaceutical Sciences, and Theory and Practice of Industrial Pharmacy, Lachman et al, 1986.

More specifically, examples of suitable surface active substances include one or combination of the following: polaxomers, such as Pluronic^{™} F68, F108 and F127, which are block copolymers of ethylene oxide and propylene oxide available from BASF, and poloxamines, such as Tetronic^{™} 908 (T908), which is a tetrafunctional block copolymer derived from sequential addition of ethylene oxide and propylene oxide to ethylene-diamine available from BASF, Triton^{™} X-200, which is an alkyl aryl polyether sulfonate, available from Rohm and Haas. Tween 20, 40, 60 and 80, which are polyoxyethylene sorbitan fatty acid esters, available from ICI Speciality Chemicals, Carbowax^{™} 3550 and 934, which are polyethylene glycols available from Union Carbide, hydroxy propylinethylcellulose, dimyristoyl phosphatidylglycerol sodium salt, sodium dodecylsulfate, sodium deoxycholate, and cetyltrimethylammonium bromide. These are all pharmaceutically acceptable surface active substances.

Preferred surface active substances are phospholipid surface active substances. By phospholipid surface active substances or phospholipid surface active agents is meant a single phospholipid or a mixture of two or more phospholipids, for example a mixture of two or three or four or five or from six to about ten phospholipids. Suitable phospholipids include animal and plant phospholipids; egg phospholipids; soya bean phospholipids; corn phospholipids; wheat germ, flax, cotton, and sunflower seed phospholipids; milk fat phospholipids; glycerophospholipids; sphingophospholipids; phosphatides; phospholipids containing fatty acid esters including palmitate, stearate, oleate, linoleate, and arachidonate which esters can be mixtures and mixtures of isomers in the phospholipids; phospholipids composed of fatty acids containing one or more than one double bonds such as dioleoyl phosphatidylcholine and egg phosphatidylcholine that are not stable as powders but are hygroscopic and can absorb moisture and become gummy; phospholipids composed of saturated fatty acids that are stable as powders and are less amenable to absorption of moisture; phosphatidylserines; phosphatidylcholines; phosphatidylethanolamines; phosphatidylinositols; phosphatidylglycerols such as dimyristoyl phosphatidylglycerol, L-alpha-dimyristoyl phosphatidylglycerol also known as 1,2-dimyristoyl-sn-glycero-3-phospho(rac-1-glycerol) and also known as DMPG; phosphatidic acid; hydrogenated natural phospholipids; and commercially available phospholipids such as those available from Avanti Polar Lipids, Inc. of Alabaster, Alabama, USA. In the absence of an internal counterion in the phospholipid, a preferred counterion is a monovalent cation such as sodium ion. The phospholipid may be salted or desalted, hydrogenated, partially hydrogenated, or unsaturated, natural, synthetic, or semisynthetic.

Preferred phospholipids include Lipoid E80, Lipoid EPC, Lipoid SPC, DMPG, Phospholipon 100H a hydrogenated soybean phosphatidylcholine, Phospholipon 90H, Lipoid SPC-3, and mixtures thereof. A currently most preferred phospholipid is Lipoid E80.

The concentration of surface active substance added to the formulations prepared as described herein can be present in the range of 0,1 to 50%, preferably 0.2 to 20%, and more preferably 0.5 to 10%. A currently preferred level of Lipoid E80 is from about 0.5% to 15%, more preferably from about 0.5% to about 10%, and most preferably from 0.5 to 5%.

Also described herein is a process for the preparation of small particles containing fenofibrate and a phospholipid surface stabilizing substance which comprises the steps of (a) mixing at high shear an admixture of the poorly water soluble drug (fenofibrate) and a phospholipid substance in an aqueous carrier in the absence of an organic solvent and optionally in the presence of one or more than one surface active substances within a first temperature range at or above the melting point of the drug to form a heated suspension containing the drug, then (b) homogenizing said heated suspension in a first pressure range and within said first temperature range to form a heated homogenate containing the drug, then (c) cooling said heated homogenate to a second temperature range below the melting temperature of the drug to form a transiently stable cooled homogenate containing the drug, then (d) applying a particle stabilizing energetic process to said transiently stable cooled homogenate within a second temperature range and in a second pressure range to form a cooled dispersion of stabilized small particles containing the drug, and then (e) optionally drying the cooled dispersion to form dried small particles containing the drug.

Also described herein is a composition and a process for the preparation of microparticles of fenofibrate, which small particles are used to prepare an orally administered pharmaceutical composition comprising said microparticles of solid fenofibrate that are stabilized by a phospholipid surface active substance, wherein said microparticles are prepared in the presence of said phospholipid surface active substance, and wherein a therapeutically effective amount of said composition provides a quantity of fenofibrate active species to a fasted human patient in need of treatment by fenofibrate that is greater than 80% of the quantity of fenofibrate active species provided by said amount to said patient when fed at least 1000 calories 50 % of which are from fat.

The process comprises the steps of (a) mixing at high shear an admixture of the poorly water soluble drug fenofibrate and a phospholipid substance in an aqueous carrier in the absence of an organic solvent and optionally in the presence of one or more than one surface active substances within a first temperature range at or above the melting point of the drug to form a heated suspension containing the drug, then (b) homogenizing said heated suspension in a first pressure range and within said first temperature range to form a heated homogenate containing the drug, then (c) cooling said heated homogenate to a second temperature range below the melting temperature of the drug to form a transiently stable cooled homogenate containing the drug, then (d) applying a particle stabilizing energetic process to said cooled homogenate within a second temperature range and in a second pressure range to form a cooled dispersion of stabilized small particles containing the drug, and then (e) optionally drying the cooled dispersion to form dried small particles containing the drug.

An admixture of a poorly water soluble drug and a surface active substances such as a phospholipid substance can be prepared by adding a surface active substance and the poorly water soluble drug to an aqueous carrier and then mixing at high shear, for example for up to 30 minutes at a shear rate of up to 10,000 rpm. As an example, an admixture of fenofibrate and a phospholipid substance can be prepared by adding a phospholipid substance and fenofibrate to an aqueous carrier and then mixing at high shear for up to 30 minutes at a shear rate of up to 10,000 rpm. Preferably the drug used to form the admixture is in the form of a powder or small crystals or small pieces that are less than about 5 mm in diameter to facilitate mixing. Larger sized crystals or masses of drug can be milled to about 5 mm or smaller before forming the admixture used as described herein to facilitate mixing.

Suitable aqueous carriers include water, sterile water, water for injection, and buffered water such as phosphate buffered water. The pH of the buffer can be in the range of from 4 to 10, preferably from 7 to 9, and most preferably from 7.5 to 8.5. A preferred aqueous carrier is 0.01 to 10 mM sodium phosphate buffer. The pH of the carrier is preferably established at room temperature before mixing with the phospholipid substance and the poorly water soluble drug and before heating to a first temperature. The pH may be adjusted by addition of an acid or base such as HCl or NaOH to a solution of a phosphate salt. Preferably the aqueous carrier contains no dissolved oxygen. A currently most preferred aqueous carder is 10 mM phosphate buffer.

In one aspect, the aqueous carrier can initially be at a temperature between about 1 °C to about 100°C; preferably between 20 °C and 90 °C, and more preferably between 20 °C and 50 °C. This is particularly useful for fenofibrate. The aqueous carrier can be heated to the desired first temperature range before or after the addition of the admixture.

In another aspect the aqueous, carrier can be heated to a temperature higher than 100 °C, for example superheated up to 275 °C. In this case, the aqueous carrier can be contained in a closed vessel or apparatus at a pressure higher than ambient pressure. The superheated aqueous carrier and the admixture can be contained in a pressurized closed system such as a stainless steel vessel or bomb in which high speed shear can be applied. The vessel is preferably connected through suitable piping and values to a heated homogenization apparatus which further comprises a reservoir and optionally a return pipe that can carry homogenate from the homogenizer back to the vessel if used in a continuous of batch-wise mode. The vapor pleasure of water at 100°C is approximately 14.7 psi and it rises as the temperature is increased. For example, at 120 °C the vapor pressure of water is approximately 28.8 psi; at 140 °C it is approximately 52.4 psi; at 1600 °C it is approximately 89.6 psi; at 180 °C it is approximately 145.4 psi; at 200 °C it is approximately 225.5 psi; at 226 °C it is approximately 337 psi; at 240 °C it is approximately 486 psi; at 260 °C it is approximately 680 psi; and at 275 °C it is approximately 863 psi. A closed system useful as described herein can safely contain the heated components described herein at least at these and higher pressures and temperatures and used to provide small particles of poorly water soluble drug as described herein.

After the poorly water soluble drug and surface active substance such as fenofibrate and a phospholipid substance are added to the aqueous carrier; the admixture can then be heated if not already so; preferably in the absence of oxygen such as under a nitrogen or argon atmosphere, until the temperature rises to a first temperature range that is at or above the melting point of the drug. In the case of fenofibrate the admixture in the aqueous carrier can be heated to between 79 °C (the reported lowest melting point of fenofibrate) and 99 °C, preferably between 79 °C and 95 °C, and most preferably between 80 °C and 90 °C. In general it is preferred that the temperature is at or up to about 20 °C above the melting point of the drug. Thus, the preferred first temperature, range is in general from the melting point of the drug to about 20 °C Above the melting point of the drug. The aqueous carrier can be heated to the first temperature range before or after the addition of the drug and the surface active substance. The admixture is maintained at the first temperature range while high shear mixing is applied. The admixture when thus prepared comprises a crude emulsion of melted drug and surface active substance in the heated aqueous carrier.

During the heating of the admixture, high shear mixing is applied. Suitable shear is derived for example from propeller-containing mixers, homogenizers, blenders, sonicators or other devices capable of producing a heated suspension. Suitable shear rates can range between 500 to 10,000 rpm, preferably 2,000 to 5,000 rpm. High shear mixing can be continued for up to 30 minutes or even longer if needed to form a heated suspension containing the drug. High shear mixing of the admixture when the temperature is below the melting point of the drug provides a suspension of the admixture in the aqueous carrier, and such suspension is useful as an antecedent to the heated suspension that is produced when the temperature is increased to or above the melting point of the drug. Continued application of high shear mixing or application of more vigorous or ultra-high shear mixing when the temperature is above the melting point of the drug can produce a heated homogenate of the admixture in the aqueous carrier. When the temperature is above the melting point of the drug, the heated suspension is a suspension of melted drug and surface active substance in the aqueous carrier. In one aspect, the heated suspension is an emulsion of melted drug and surface active substance in the aqueous carrier. High shear mixing and ultra-high shear mixing can be produced by the input of mechanical energy for example using a mechanical mixer or stirrer or mill configured with a mixing blade or propeller that can induce efficient mixing and particle size reduction through high shear turbulence, turbulent eddies, transfer of high fluid kinetic energy, high energy dissipation, pressure induced cavitation, and similar known mechanisms of homogenization.

In one aspect, devices useful in the preparation of a heated suspension described herein can be employed in the preparation of the heated homogenate described herein if sufficient energy is transferred to the particles of the heated suspension to produce a heated homogenate. In this case, heating of the admixture to form a heated suspension and then homogenization of the heated suspension to form a heated homogenate can be done as a continuous step combining step (a) and step (b) into a single step wherein a heated suspension is formed and then converted into a heated homogenate without substantial change in apparatus or without substantial increase in energy applied to the heated admixture formulation.

As used herein, homogenization refers to the creation of a homogenate or uniform distribution of small particles containing drug in an aqueous carrier as a result of an energetic process being applied to an antecedent composition such as a mixture, admixture, blend, emulsion, suspension, dispersion or other composition of solids or solid particles or liquids or liquid particles or droplets comprising drug and one or more than one surface active substance In an aqueous carrier wherein the homogenate and the small particles produced are at least transiently stable toward phase reparation into larger particles or droplets or non-uniform solid or liquid domains. Homogenization, particularly with respect to the formation of a heated suspension and a heated homogenate, can be achieved by input of mechanical energy such as by high shear mixing, ultra high shear mixing, high speed blending, microfluidization, and nulling such as by dispersion milling, ball milling, attritor milling, vibrator milling and media milling, or by application of sonic energy in the form of sonication. Preferably in the case of a mill being used in this process wherein the mill contains media or grinding media, such media is removed in a filtration or other suitable separation process to provide homogenized compositions as described herein. Homogenization is preferably achieved by passing an antecedent composition under high pressure, for example under more than 1000 psi, through a tiny orifice which can result in a decrease in the average diameter and an increase in the number and surface area of particles or droplets in the antecedent composition and produce small particles. A preferred homogenization method comprises passing an antecedent composition under high pressure through a tiny orifice and includes microfluidization, particularly with respect to homogenization to prepare a cooled dispersion are described herein.

The drug can be added to the aqueous carrier as at solid. Preferably for example the drug such as fenofibrate can be added in the form of particles ranging in size up to about 10 mm such as milled or micronized particles or powders. Milled particles can be obtained for example by air jet milling of bulk powdered or crystalline fenofibrate. The drug can also be added to the aqueous carrier as a molten material, i.e., heated at or above its melting point, preferably at the melting point of the drug to about 20 °C above the melting point of the drug but at a temperature less than its decomposition point. For fenofibrate the preferred. temperature can be from about 80 °C, the melting point of the drug, to about 100 °C although temperatures up to the decomposition point of the drug are also suitable.

The concentration of the surface active substance in the aqueous carrier can vary between 1% w/w and 90% w/w, preferably between 0.1% w/w and 50% w/w, and more preferably between 0.2% and 20%, and most preferably between 0.5% to 10% w/w. The concentration of the drug such as fenofibrate in the aqueous carrier can vary between 0.1% w/w and 90% w/w, preferably between 0.5% w/w and 50% w/w, and more preferably between 1% and 20% w/w. For example, in one aspect a currently preferred composition comprises 3% to 10% of a phospholipid substance as a surface active substance and 10% of the poorly water soluble drug fenofibrate in 10 mM phosphate buffer at pH 8 as an aqueous carrier. Another currently preferred composition comprises 0.5% of a phospholipid substance as a surface active substance and 10% of the poorly water soluble drug fenofibrate in 10 mM phosphate buffer at pH 8 as an aqueous carrier. Another currently preferred composition comprises 1.5% of a phospholipid substance as a surface active substance and 10% of the poorly water soluble drug fenofibrate in 10 mM phosphate buffer at pH 8 as an aqueous carrier.

The surface active substance can be added to the aqueous carrier at any temperature below its decomposition point. When used as a mixture of surface active substances, the individual components can be added separately to the aqueous carrier or combined as mixtures before addition. The surface active substance can be added together with the drug, for example with fenofibrate or separately to the aqueous carrier.

The admixture of the drug, for example fenofibrate, and a surface active substance such as a phospholipid substance in an aqueous carrier is heated to a first temperature range during the application of a high shear mixing to produce a heated suspension containing the drug.

The heated suspension containing the drug is then homogenized at the first temperature range to form a heated homogenate. The first temperature range is maintained during this homogenization to ensure that the drug is maintained in a molten state. For fenofibrate, the first temperature range is preferably from 79 °C to 100 °C and more preferably from 80 °C to 100 °C provided that fenofibrate remains molten.

Homogenization of the heated suspension containing the drug can be carried out in equipment suitable for that process. Useful equipment includes commercially available high pressure homogenization equipment such as APV Gaulin M15, Avestin Emulsiflex C5 or C50, and MFIC Microfluidizer M110EH and other commercially available microfluidizers and commercially available microfluidizers modified to accommodate heat exchangers and temperature monitoring devices and piping and valves to carry heated suspensions or emulsions. The microfluidizers can be heated to the first temperature range, for example by use of electrical resistance, heated air bath, or heated fluid bath such as a water or silicone oil bath heated to the first temperature range that is at or above the melting point of the drug.

Homogenization of the heated suspension containing the drug is done at a first pressure range in the homogenization chamber of a heated homogenization apparatus while the drug is maintained in its molten state. The first pressure range can be from 2,000 psi to 30,000 psi, preferably about 5,000 psi to 20,000 psi, and more preferably from about 3,000 psi to about 10,000 psi.

The heated suspension containing the drug can be processed into the homogenization chamber of the homogenization apparatus by gravity feed from a heated and optionally stirred reservoir or by aid of a pump, for example a peristaltic pump, from a reservoir heated to the first temperature range through the heated Homogenization chamber of the heated homogenizer and thence into a heated receiving vessel heated to the first temperature range in such a manner as to ensure the entire fluid volume of the heated suspension is subjected to discrete homogenization resulting in a homogeneous suspension of heated submicron or micron molten particles. In one aspect of this disclosure, between each homogenization pass the processed heated suspension is returned batch-wise from the heated receiving vessel back into the heated reservoir such as by means of a pump or by pouring and the heated homogenization step is repeated. In another aspect, the processed heated suspension is fed directly back into the heated reservoir in a continuous process. If the aqueous carrier is heated above 100°C, the system is contained as a closed system under pressure during the feeding of the admixture to the homogenization apparatus and during the return of the homogenized or partially or not-completely homogenized heated suspension to the heated reservoir. If the initial volume of the heated suspension before homogenization is defined as a volume pass, then the number of Volume passes made through the homogenizer in this manner can range from one to abort 20, preferably from one to ten, more preferably from 2 to 8, and most preferably from 3 to 7 to produce a heated homogenate that is initially at the first temperature range at or above the melting point of the drug. A preferred drug in this process is fenofibrate which has a preferred first temperature range of from 80 °C to about 95 °C.

While it is not known with certainty, it is appreciated that forcing a drug and a surface active substance such as a phospholipid under conditions of elevated pressure and temperature through a microfluidizing chamber can cause transient gradients in temperature, the' microfluidization process being exothermic and causing a rise in the temperature of the processed suspension of particles or emulsions during particle size reduction. While the transient rise in temperature is usually controlled by a temperature regulating device such as a heat exchanger, it is possible that transient concentration gradients of poorly water soluble drug and stabilizer are established or continue to exist in the rapidly moving non-equilibiium state of the microfluidizer. Water insoluble or poorly soluble components of the formulation (e.g., fenofibrate and phospholipid) may be forced into solution temporarily, perhaps at a molecular level thereby creating a supersaturated or molecularly distorted environment which if left undisturbed will subsequently achieve equilibrium again. It is postulated that transient concentration gradients may be established in the microfluidization process wherein molecules of drug and stabilizer are forced into an aqueous environment to give a transiently stable but novel composition and non-equilibrium condition.

We have found that this heated homogenate can be cooled to a transiently stable or metastable cooled homogenate. By metastable we mean that upon agitation or long-term standing the transiently stable particles of the cooled homogenate will convert to larger particles of crystallized or precipitated drug and can demonstrate phase separation of components of the homogenate from the aqueous carrier. For example, under these conditions fenofibrate forms a transiently stable or metastable cooled homogenate that on standing or application of manual agitation such as shaking or stirring produces larger crystals. However, we have surprisingly found that the lifetime of the transiently stable particles of the cooled homogenate can be moderately extended by control of cooling conditions. Additional prolonged stability of the small particles can be obtained by subsequent homogenization at a second temperature range that is below the melting point of the drug. We have also found that the total number of homogenization volume passes used in the heated and cooled homogenization processes described herein is substantially fewer than the number of volume passes needed to produced a comparable drug particle size suspension starting from the powdered or micronized drug that was used to prepared the admixture described herein but homogenized while the drug was maintained entirely in the solid state according to prior art methods.

In one aspect particle size of the heated homogenate can be measured using a laser light diffraction based instrument such as a Malvern Mastersizer Microplus and shown to be less than one micrometer.

If an attempt is made to collect the heated homogenate in a receiving vessel that is not preheated to the first temperature, a poorly water soluble drug such as fenofibrate immediately precipitates from the heated homogenate as a solid; and in the case of fenofibrate as crystals. This is very likely related to agitation of the transiently stable dispersion.

In the case of fenofibrate, microscopic examination of a heated homogenate shows it to' be comprised of small and non-crystalline particles in suspension, but there is a tendency for fenofibrate to crystallize out on the microscope slide. This rapid crystallization is also seen if the heated homogenate is collected in a receiver at ambient temperature.

A transiently stable or metastable cooled homogenate can be obtained from a heated homogenate derived from an admixture of drug and a surface active substance such as a phospholipid substance in an aqueous carrier by rapidly cooling the heated homogenate under non-agitating conditions from a first temperature range at or above the melting temperature of the drug to a second temperature range below the melting point of the drug, preferably to the range of 1 °C to about 20 °C. In some cases, depending on how readily the drug crystallizes, under non-stirred conditions the cooled homogenate can retain small non-crystalline particles very similar to those detected initially in the heated homogenate. Optionally, the heated homogenate can be held at the first temperature range that is above the melting point of the drug, for a holding time before the onset of cooling to the second temperature range. Agitation during the holding period above the melting point of the drug does not effect crystallization of the drug. However, agitation such as by stirring of the cooled homogenate can induce growth in particle size and crystallization and precipitation of drug.

In particular, in the case of fenofibrate we have found that a transiently stable or metastable cooled homogenate can be obtained from a heated homogenate derived from an admixture of fenofibrate and a phospholipid substance in an aqueous carrier by rapidly cooling the heated homogenate under non-agitating conditions from a first temperature range at or above the melting temperature of fenofibrate to a second temperature range below the melting point of fenofibrate, preferably to the range of 1 °C to about 20 °C. Under non-stirred conditions the cooled homogenate retains small non-crystalline particles very similar to those detected initially in the heated homogenate. Optionally, the heated homogenate can be held at the first temperature range, for example at 80 °C to 90 °C, for a holding time before the onset of cooling to the second temperature range. Agitation during the holding period does not effect crystallization of the fenofibrate.

To determine a minimum holding time at 80 to 90 °C before the induction of cooling for a fenofibrate-containing heated homogenate, the holding time was varied at 15 minute intervals from 0 to 60 minutes and a cooling period in a bath held at 5°C was kept constant at 30 minutes after the onset of cooling. In these experiments we find that particle mean diameters of the cooled homogenate are similar under all conditions studied. Thus, samples of freshly prepared heated homogenate can be held at a first temperature range for a holding period or they can be immediately cooled to a second temperature range after completion of the first homogenization step.

A number of cooling methods can be applied to the heated homogenate containing a poorly water soluble drug to cool it from the first temperature range at or above the melting point of the drug to a temperature below the melting point of the drug to form a cooled homogenate. Examples of several methods are listed and illustrated with respect to fenofibrate as follows.
Method 1: slow cooling in ambient air optionally in a closed vessel that excludes oxygen and air by allowing the heated homogenate to stand unagitated and to cool from above the melting point of the drug to ambient room temperature;
Method 2: slow unagitated cooling from above the melting point of the drug which for fenofibrate is about 85 °C in a water bath at ambient temperature which is approximately 15 **°C** to 20 °C
Method 3 slow stepwise cooling at 1 degree Centigrade per minute in a stirred oil bath from above the melting point of the drug to ambient temperature
Method 4: slow stepwise cooling from above the melting point of the drug to about 20 °C below the melting point of the drug which for fenofibrate is from about 85 °C down to 65 °C, followed by cooling to 4 °C in an isothermally cooled 4 °C water bath;
Method 5: fast cooling in an isothermally cooled 4 °C water bath;
Method 6: slow stepwise cooling from above the melting point of the drug to about 40 °C below the melting point of the drug which for fenofibrate is from about 85 °C to about 40 °C at the rate of 1 Centigrade degree per minute**.**

For cooling from temperatures initially above 100 °C the heated homogenate is maintained in a pressurized vessel. After cooling, the pressure can then be optionally adjusted to ambient without agitation of the contents of the vessel typically by means of a valve that permits pressure equalization to ambient pressure conditions. Preferably an inert atmosphere such as a nitrogen or argon atmosphere is maintained in contact with the formulations described herein.

The effect of stirring during the cooling phase was examined for fenofibrate as an example. In some studies, samples were left unagitated while others were stirred magnetically at 250 rpm using Teflon-coated magnetic stirring bars during cooling methods. Additionally, in some experiments, heated homogenate was diluted ten fold with additional aqueous carrier that had been heated to the first temperature, the diluted heated homogenate was then swirled to evenly distribute the added aqueous carrier, and then the diluted heated homogenate was cooled.

Particle size determinations were carried out using a Malvem Microplus Mastersizer. Samples were examined at two to three hours after the initiation of cooling. Results are reported as volume weighted averages or D(4,3). Samples were also examined microscopically under bright polarized light using both in-phase and out-of-phase modes. In-phase light allowed determination of the primary particle- size and the detection of aggregates. Out-of-phase examination gave an indication of the amount of crystals formed in the composition. Morphologically small crystalline particles of fenofibrate were easily distinguished from large fenofibrate crystals.

When 3 % Lipoid E80 (also sometimes referred to as E80 herein below) was used as a phospholipid substance in a single pass homogenization preparation of a heated homogenate containing 10 % fenofibrate, little difference was observed in the particle characteristics when cooled by either method 1 or 2 (average particle size at 3 hours was 2.42 and 2.96 micrometers, respectively). The particles were initially non-crystalline, spherical and submicron but crystals appeared within 3 hours. In contrast, when 3 % Lipoid E80 was used as a phospholipid substance in a two pass homogenization preparation of a heated homogenate containing 10 % fenofibrate, a smaller particle size was unexpectedly observed when a sample was cooled by method 1 versus when a sample was cooled by method 2 (0.56 and 1.64 micrometers, respectively after 3 hours of cooling). This difference was different from that seen in heated homogenates prepared with saturated lipids such as phospholipon 100H (also sometimes referred to as 100H herein below) and phospholipon 90H (also sometimes referred to as 90H herein below) when processed for two passes. In these formulations, the particle size at 2 to 3 hours after initiation of cooling was significantly higher than that seen using Lipoid E80. For heated homogenates prepared using 3% phospholipon 100H in two passes and cooled for 3 hours according to methods 1 and 2, the average particle sizes were 14.72 and 10.31 micrometers, respectively. For heated homogenates prepared using 3% phospholipon 90H in two passes and cooled for 2 hours according to methods 1 and 2, the average particle sizes were 6.07 and 5.23 micrometers, respectively. Microscopically the cooled homogenates containing phospholipon 100H and phospholipon 90H consisted of particle aggregates with crystals appearing over time. Aggregates were not typically seen in Lipoid E80 formulations but crystal growth occurred over time.

It was unexpectedly found that increasing the cooling rate in the absence of agitation produced cooled homogenates that maintained small particles containing the poorly water soluble drug fenofibrate to a greater degree than those produced by slow cooling methods. This was especially true when Lipoid E80 was used as the phospholipid substance. For example, when a sample of heated homogenate prepared from 3% Lipoid E80 as the surface active substance and 10% fenofibrate in two homogenization passes was cooled by method 5 (fast cooling) and compared to a cooled sample of heated homogenate of the same composition cooled according to methods 1 or 2 (slow cooling), the particle size at 3 hours for fast cooling was 0.63 micrometers versus 0.76 micrometers for slow cooling.

For non-stirred samples, minimal particle size increases can be observed in all cooling methods while under stirred conditions substantial crystallization or precipitation or agglomeration of poorly water soluble drug can be observed. For example, for non-stirred samples containing fenofibrate, minimal particle size increases were observed in all cooling methods. In contrast, under stirred conditions substantial crystallization of fenofibrate was observed for all cooling methods. For samples cooled in a slow step process, crystal growth occurred at temperatures lower than about 20 °C below the melting point of the drug, i.e., for fenofibrate below about 60 °C.

It can be seen that energy imparted to the cooled homogenate by mechanical stirring for example using a stirring bar or spatula is not sufficient to impart stability to the particles of the cooled homogenate. To be effective, a particle stabilizing energetic process must impart sufficient energy to the particles of the cooled homogenate to convert them from a transiently stable homogenate into a longer lived dispersion of particles. Otherwise, undesirably large particles will be produced from the transiently stable cooled homogenate. Preferred particle stabilizing energetic processes include sonication and homogenization. A most preferred particle stabilizing energetic process is homogenization. It is believed that enough energy must be applied to the particles to modify some aspect of the particle composition which, while currently unknown, may be related to further reduction in particle size in the presence of a surface active substance or reorganization of drug and/or surface active substance molecules at or on the surface of the particle, or other phenomena.

Oral formulations of fenofibrate microparticles stabilized by phospholipid surface active substance and prepared by homogenization or microfluidization or hot melt homogenization or sonication provide unexpected reduction in food effect on the uptake of fenofibrate between fasted and fed conditions.

Diluting the heated homogenate ten fold with additional heated aqueous carrier was found unexpectedly to have a beneficial effect on the size of particles when cooled. Results for fenofibrate as an example are displayed in Table 2. Attention is drawn to the bottom two rows of Table 2 which show that the particle size of diluted suspension of fenofibrate is smaller than that of undiluted suspension.

| Table 2. Effect of dilution with aqueous carrier on cooled particle sizes in micrometers of heated homogenate containing 10% fenofibrate and 3% phospholipid | | | | | | |
|---|---|---|---|---|---|---|
| Phospholipid | E80 | E80 | 100H | 100H | 90H | 90H |
| (one pass) | | | | | | |
| Cooling method | 1 | 2 | 1 | 2 | 1 | 2 |
| (time of cooling) | (3h) | (3h) | (3h) | (3h) | (2h) | (2h) |
| Undiluted average | 2.42 | 2.96 | 11.46 | 9.71 | 4.83 | 4.12 |
| particle size | | | | | | |
| Diluted average | 1.84 | 1.69 | 3.29 | 3.77 | 2.17 | 2.73 |
| particle size | | | | | | |

Cooled homogenate having particle size of less than 1 micrometer can usually be achieved by subjecting the heated homogenate containing melted drug to multiple homogenization passes prior to rapid cooling. The effect of multiple homogenization is to produce smaller particles, but the size reducing effect is non-linear and shows decreasing rates of return, i.e., the average particle size decreases non-linearly with an increasing number of passes.

In the case of fenofibrate, it was also found that increasing the number of heated homogenization passes from one to two followed by cooling produced a cooled homogenate with smaller particle size with Lipoid E80 but not with Phospholipon 100H or Phospholipon 90H. For example, at 3 hours after cooling, a cooled homogenate sample containing fenofibrate prepared according to method 1 had a particle size of 0.56 micrometers when the antecedent heated homogenate had been subjected to two passes of homogenization compared to a particle size of 2.42 micrometers when the antecedent heated homogenate had been subjected to one homogenization pass. When a heated homogenate had been subjected to 10 homogenization passes, the cooled homogenate had a particle size of 0.29 micrometers. It was generally found that cooled homogenate having particle size of about 0.3 micrometers could be achieved from heated homogenate that had been subjected to at least 5 homogenization passes. Additional homogenization produced smaller particles, but at decreasing rates per volume pass. For examples, particles as small as 0.05 micrometers can be achieved under homogenization conditions. Results for one and two homogenization volume passes as a function of phospholipid are displayed in Table 3.

| Table 3. Difference between one and two heated homogenization passes on cooled particle sizes in micrometers of heated homogenates containing 10% fenofibrate and 3% phospholipid | | | | | | |
|---|---|---|---|---|---|---|
| Phospholipid | E80 | E80 | 100H | 100H | 90H | 90H |
| (no. of passes) | | | | | | |
| Cooling method (time | 1 | 2 | 1 | 2 | 1 | 2 |
| of cooling) | (3h) | (3h) | (3h) | (3h) | (2h) | (2h) |
| One pass average | 2.42 | 2.96 | 11.46 | 9.71 | 4.83 | 4.12 |
| particle size | | | | | | |
| Two pass average | 0.56 | 1.64 | 14.72 | 10.31 | 6.07 | 5.23 |
| particle size | | | | | | |

We have also found that the pass dependent particle size of the cooled homogenate can be a function of the ratio of the concentration of surface active substance to drug. For example, a heated homogenate prepared using 3 % Lipoid E80 as the surface active substance and 10 % fenofibrate as the drug and subjected to 10 homogenization passes produced a cooled homogenate by method 6 that had a particle size of 0.35 micrometers while a heated homogenate prepared using 10 % Lipoid E80 as the surface active substance and 10 % fenofibrate as the drug and subjected to 10 homogenization passes produced a cooled homogenate by method 6 that had a particle size of 1.3 micrometers.

Furthermore, when a heated homogenate was prepared using 3 % Phospholipon 100H as the surface active substance and 10 % fenofibrate as the drug, subjected to 10 homogenization passes and cooled, a cooled homogenate was produced by method 5 that had a particle size of 1.45 micrometers. In comparison, when a heated homogenate was prepared using 3 % Lipoid E80 as the surface active substance and 10 % fenofibrate as the drug, subjected to 10 homogenization passes and cooled ,a cooled homogenate was produced that had a particle size of 1.3 micrometers.

Fast cooling of heated homogenates in a 4 °C bath under non-stirred conditions produces cooled homogenates with minimum change in morphology and particle size from that observed in the heated homogenates prior to cooling. For example, we have discovered that fast cooling of heated homogenates containing a phospholipid as the surface active substance and fenofibrate as the drug in a 4 °C bath under non-stirred conditions produced non-crystalline cooled homogenates with minimum change in morphology and particle size from that observed in the heated homogenates prior to cooling. When samples of heated homogenate were held at 80 °C for up to one hour and then cooled to form cooled homogenates that were held for 30 minutes at 5 °C, no differences in particle size could be detected as a function of the time the heated homogenate was held at 80 °C before cooling. For optimum processing speed, freshly prepared samples of heated homogenate can be cooled from the first temperature range to a second temperature range immediately after an adequate number of homogenization passes such as five passes of heated homogenization to provide cooled homogenates. However, cooled homogenates thus prepared appear to be transiently stable or metastable toward Formation of crystals of drug that can grow larger and precipitate from the suspension of the cooled homogenate if allowed to stand. The formation of larger particles and crystals is enhanced if the cooled homogenate is disturbed such as by stirring or shaking.

Preferably, the average particle size of the microparticles of fenofibrate stabilized with phospholipid is less than 10 microns, more preferably less than 5 microns, even more preferably less than 4 microns, yet even more preferably less than 3 microns, yet even more preferably less than 2 microns, and most preferably less than 1 micron. Microparticles that are less than about 0.5 microns are especially preferred.

Bulking agents or bulking agent excipients can be added as solids or in solutions of aqueous carrier to the admixture of drug and a surface active substance in an aqueous carrier in the process described herein.

A bulking agent is herein defined as compound useful in assisting redispersion of dried small particles back into a suspension such as an aqueous suspension. Suitable bulking agents include hydroxyl-containing, hydrophilic, relatively low molecular weight (less than 50,000) compounds such as monosaccharides, disaccharides**,** trisaccharides, sucrose, raffinose, lactose, mannitol, sorbitol, trehalose, glycerol, dextrose, maltodextrose, fructose, sugars, pentoses, hexoses, xylitol, and mixtures thereof.

Optionally, bulking agents can include one or more amino acids, preferably naturally occurring or essential amino acids, proteins, peptides, vitamins such as vitamin A, vitamin C (ascorbic acid), citric acid, cellulose and modified cellulose acceptable for pharmaceutical or food use such as carboxymethylcellulose and salts thereof, albumin, aspartame, povidone, crospovidone, croscarmellose sodium (Ac-Di-Sol) and related salts, additional phospholipid such as egg lecithin, magnesium salts such as magnesium stearate, magnesium carbonate, magnesium aluminum silicate, magnesium trisilicate, maltodextin,- polyethylene glycol**,** pluronic surfactants, polyethylene glycol esters acceptable for pharmaceutical use, polyethylene glycol ethers acceptable for pharmaceutical use, polymethacrylates acceptable for pharmaceutical use, polyvinyl alcohol acceptable for pharmaceutical use, polyvinyl acetate and partially hydrolyzed polyvinyl acetate acceptable for pharmaceutical use, saccharin, sodium saccharin, potassium sorbate, silicon dioxide, sodium lauryl sulfate, sorbitol, starch and modified starch, pharmaceutically acceptable organic acids such as stearic acid, palmitic acid, tartaric acid, sorbic acid, fumaric acid, alginic acid, lactic acid, edetic acid, and pharmaceutically acceptable salts thereof, pharmaceutically acceptable flavorings. pharmaceutilly acceptable coloring agents, and other pharmaceutically acceptable excipients such as pharmaceutically acceptable diglycerides and triglycerides, pharmaceutically acceptable fatty acids such as oleic acid, stearic acid, palmitic acid, and myristic acid, fatty acid sorbitan esters, Tween surfactants, PEG-castor oil surfactants, omega-3-fatty acids and their salts, and mixtures thereof. These bulking agents can be added in amounts from about 0.5% to about 60% by weight to a dried powder and then formed into tablets or capsules or powders or granular dosage forms.

In another aspect, bulkind agents such as those listed herein can be added as excipients to the stabilized particles described herein either in suspension or as dried powders and then blended and formed into dosage forms such tablets, capsules, powders, and suspension of particles.

In one aspect, bulking agents are useful as protectants in a drying process such as cryoprotectants in a lyophilization process or as additives in a spray drying process or an-evaporation process, preventing or substantially reducing particle fusion, combination, suspension degradation and agglomeration during drying, and assisting in the resuspension of particles from a dried state. Dry small particles containing a poorly water soluble drug can be produced for example as a dried lyophilizate which is a solid produced from a cooled dispersion of particles by the process of freezing the aqueous carrier to a solid comprising a dispersion in ice and then removing the water by subliming the ice under reduced pressure. Bulking agents can also reduce or depress the freezing point of aqueous compositions in which they are dissolved or partially dissolved. Bulking agents can also facilitate the redispersion of the phospholipid stabilized particles by dissolving or hydrating and freeing the particles, particularly in the gastrointestial tract of a person who has swallowed a dosage form of fenofibrate for use in this invention.

Bulking agents can be added in amounts from 0.1% to about 50% w/w or more depending on the intended use. Additional amounts of bulking agents can be added to the phospholipid-stabilized microparticles after they have been prepared as a suspension, for example prior to a drying step such as a spray drying step or a lyophilization step, or after they have been dried or substantially dried. Mixing of bulking agents to dried or substantially dried microparticles can be done by mixing the ingredients or by adding one or more bulking agents to the microparticles or vice versa and subsequently blending the ingredient. Alternatively, the microparticles can be resuspended in a liquid or fluid such as an aqueous fluid and admixed with bulking agents as solutions, suspensions, or, as dried substances, and the liquid or fluid can be subsequently removed. Depending on the intended use and ultimate formulation and dosage form, bulking agents such as monosaccharides, disaccharides, trisaccharides, sucrose, raffinose, lactose, mannitol, sorbitol, trehalose, glycerol, dextrose, maltodextrose, fructose, sugars, pentoses, hexoses, xylitol, and mixtures thereof can be added in amounts varying from about 0.1% up to their solubility limits in this aspect. A preferred range of these ingredients is such to provide from about 1% to about 90% of a tablet or capsule dosage form. A preferred range for the active ingredient, a fibrate such as fenofibrate in a tablet form 10% to about 90% by weight of the tablet, with a more preferred range being from about 15% to about 60%.

As described herein the phospholipid-stabilized microparticles can be sprayed onto the surface of a bulking agent, for example if the bulking agent is in the form of a particle or bead, a suspension of phospholipid-stabilized microparticles optionally containing dissolved or suspended bulking agent can be spray coated onto the surface of the bulking agent particle or bead to create a layer and optionally a multilayer derived from repetitive spray coating.

Preferred bulking agents include mannitol, trehalose, sucrose, sorbitol, and mixtures thereof. Preferred levels of these bulking agents in the admixture range from about 1% to about 30% w/w, and more preferably from about 2% to about 25% w/w.

The phospholipid-stabilized microparticles that exhibit a substantial reduction in food effect as described in this invention can be employed in a number of dosage forms. Particularly useful are the dosage forms disclosed in WO 00/30616.

Bulking agents can be added to the admixture, to the heated suspension, to the heated homogenate, to the cooled homogenate to the cooled dispersion, and to the dried particles. They can be added as solids or as liquids or as solutions in aqueous carrier.

The stability of cooled homogenate formulations with respect to the effect of addition of a bulking agent or a combination of bulking agents was examined. When bulking agents were added as solids or liquids to heated admixtures of fenofibrate and a phospholipid substance as a surface active substance in an aqueous carrier, then processed for example using 10 heated homogenization passes at 80°C and subsequently cooled in a 4 °C water bath, particle size estimates suggested that with the exception of the bulking agent sucrose (10%), there was little increase in particle mean diameter measurements over a 2h period. However microscopic observations revealed the presence of a significant number of large crystals after the cooling step. Addition of 2-fold hot buffer solution containing either nothing or bulking agents to the processed formulations caused a large increase in the mean particle diameter. This was attributed by microscopic examination to be due to particle aggregation together with large crystals also present.

When trehalose was added to an admixture of fenofibrate and a phospholipid substance in an aqueous carrier, on stirring crystals were detected indicating that trehalose did not stabilise these metastable formulations with respect to crystal formation and precipitation. PVP 17 and glycerol were added to heated homogenates, and in both cases crystal growth was observed microscopically under stirred conditions. When glycerol alone or glycerol and trehalose were added to the admixture and then homogenized, results from stirring experiments again showed that these formulations were unstable with extensive crystallization observed over time. Thus, adding bulking agents or PVP to either the admixture or to the heated homogenate does not result in stabilization of the metastable formulation under stirring conditions.

Whereas a cooled homogenate can be unstable with respect to agitation such as stirring or manual shaking, we have surprisingly found that a cooled homogenate can be transformed into a more stable cooled dispersion by application of a particle stabilizing energetic process applied at the second temperature range and in a second pressure range.

For example, although the aforementioned cooled homogenates of fenofibrate were found to be unstable with respect to agitation such as stirring or manual shaking that lead to the formation of crystals of fenofibrate, we have found that the cooled homogenate can be transformed into a more stable cooled dispersion by application of a particle stabilizing energetic process applied at the second temperature range and in a second pressure range.

Examples of suitable particle stabilizing energetic processes include homogenization, microfluidization, and sonication. Microfluidization is generally considered to be a method of homogenization. Microfluidization of fenofibrate in the presence of a phospholipid stabilizing agent produces a novel composition that when formulated into a suitable dosage form as a dried solid optionally in the presence of one or more excipients such as sucrose, sorbitol, trehalose, Tween 80, mannitol, other sugars and starch, and the like provides a novel oral dosage form of the drug which when taken by a fasting or a fed patient exhibits a differential uptake of the drug by the fasted patient of at least 80% of the AUC amount of drug taken up by a patient fed a high fat meal. The unexpected and sizable reduction in food effect on the uptake of drug by fasted and fed patients is useful in the prescription of the drug to a patient undergoing treatment in that the patient will receive comparable and therapeutically useful levels of the drug regardless of whether the patient is fed or fasted.

The mechanism of obviation of food effect in a patient taking the dosage form of fibrate in this invention is not yet fully understood, but it can be postulated that the phospholipid is uniquely involved in several aspects that lead to this novel discovery. For example, the phospholipid is involved in the stabilization of the fibrate particles during their formation and manipulation during formation of the dosage form; the phospholipid is involved in the reconstitution and continued stabilization of the particles during disintegration of the oral dosage form in vivo; and the phospholipid is perhaps involved in a mechanism leading to dissolution of the particles in vivo and/or uptake of the drug into the blood, e.g., molecular association between phospholipid and drug and other in vivo substance in some sort of transport mechanism.

In one aspect, particles of a heated homogenate containing a poorly soluble drug can be non-crystalline while the cooled dispersion particles produced as a result of application of a particle stabilizing energetic process can be crystalline. While stirring can induce significant particle growth in a cooled homogenate, stirring does not induce significant particle growth in a cooled dispersion formed from the cooled homogenate. The cooled dispersion thus produced is more robust toward particle growth than the cooled homogenate. The particles of the cooled dispersion are preferably in the micron and submicron range. Depending on the number of stabilizing processing steps, i.e., volume passes, employed in the preparation of the cooled dispersion, the cooled dispersion can also comprise weakly associated aggregates of particles that can be readily broken up or dispersed or de-aggregated by stirring the dispersion. Preferably, an increase in the number of processing steps from 1 to a range of from 5 to 20, preferably from 10 to 20, can produce fewer and more easily dispersed aggregates. Formulation instability toward stirring can be increased as a result of the particle stabilizing energizing process.

Microscopically, in the case of fenofibrate as an example of a poorly soluble drug, heated homogenate particles are non-crystalline while cooled dispersion particles produced as a result of application of a particle stabilizing energetic process are crystalline. Importantly, while stirring can induce significant particle growth in a cooled homogenate, stirring does not induce significant particle growth in a cooled dispersion formed from the cooled homogenate. The cooled dispersion thus produced is more robust toward particle size growth than the cooled homogenate. One possible explanation is that the number of nucleation sites for formation of crystals of the poorly soluble drug is substantially increased by application of a particle stabilizing energetic process such as microfluidization in the presence of a surface active substance giving rise to stable small crystalline particles in the micron and submicron range.

A preferred particle stabilizing energetic process is microfluidization for example using a Microfluidix M110EH apparatus. Microfluidization can be accomplished using from 1 to 20 volume passes, preferably from 2 to 20 volume passes, more preferably from 5 to 20 volume passes, and most preferably from 10 to 20 volume passes. Microfluidization can be done in continuous mode or in batch mode. A preferred second temperature range is the second temperature range used for the preparation of the cooled homogenate and is preferably from 1 °C to 40 °C, more preferably form 4 °C to 20 °C and most preferably from 4 °C to 15 °C. A useful pressure range for the preparation of the cooled dispersion is a second pressure range, that is, from 2,000 to about 30,000 psi, preferably from 5,000 to about 20,000 psi, and most preferably from 5,000 to 18,000 psi.

Microscopically, in the case of fenofibrate as an example, the cooled dispersion is a suspension of crystalline fenofibrate particles. Depending directly on the number of stabilizing processing steps or volume passes employed in the preparation of the cooled dispersion, the cooled dispersion can also comprise weakly associated aggregates of crystalline fenofibrate particles that can be broken up or dispersed or de-aggregated by stirring the suspension.

Figure 1 is an optical microscopic comparison of microfluidized fenofibrate with micronized fenofibrate and fenofibrate compositions prepared in the presence of starch. In Figure 1(A), crystals of fenofibrate 20 and domains of starch 10 are large with respect to the 100 micrometer scale. In Figure 1(B), encircled micronized fenofibrate 40 is seen to be non uniformly sized and dispersed and particles are entrained in starch domain 30. In Figure 1(C), encircled microfluidized fenofibrate particles 40 that are stabilized with phospholipid are uniformly distributed in an average size smaller than micronized fenofibrate of Figure 1(B).

A reduction in the cooled dispersion particle mean diameter can be achieved by increasing the number of volume passes during the cold homogenization step. For example, as shown in Table 4 for a formulation derived from an admixture of 3% Lipoid E80 as the surface active substance and 10% fenofibrate as a poorly water soluble drug processed first for 10 volume passes to form a heated homogenate containing the drug, cooled according to method 5 to form a transiently stable cooled homogenate containing the drug, and then microfluidized for 2 volume to 10 volume passes to form a cooled dispersion of small particles containing the drug, the observed mean diameter was 0.26 to 0.54 micrometers as a cooled homogenate prior to undergoing a particle stabilizing energizing process, 1.45 micrometers as a cooled dispersion when processed for 2 volume passes, and 0.9 micrometers when processed for 10 volume passes. Surprisingly, formulation instability toward stirring was dramatically increased as a result of the particle stabilizing energizing process. Without the additional particle stabilizing energizing process, the average particle size of the cooled homogenate increased by two orders of magnitude with stirring within 30 minutes. However, after application of the particle stabilizing energizing process, the average particle size did not increase substantially with stirring up to 24 hours. In addition, the average particle size of the cooled dispersion was smaller and remained smaller up to 5 days when the formulation was processed for 10 volume passes.

| Table 4. Particle size changes of cooled homogenate and dispersion | | | |
|---|---|---|---|
| From an admixture of 10 % Fenofibrate, 3 % Lipoid E80 as the surface active substance in 10 mM phosphate buffer at pH 8. Keeping temperature was 4°C. | | | |
| | Time (minutes) | Average size not stirred (micrometers) | Average size stirred (micrometers) |
| Cooled homogenate (10 volume Passes) | 0 | 0.26 | 0.26 |
| | 30 | 0.26 | 14.22 |
| | 60 | 0.54 | 9.44 |
| | | | |
| Cooled dispersion | 0 | 1.45 | 1.45 |
| (2 volume Passes) | | | |
| | 30 | 1.45 | 1.29 |
| | 60 | 1.37 | 1.37 |
| | 1440 | Not measured | 1.12 |
| | | | |
| Cooled dispersion (10 volume passes) | 0 | 0.87 | Not measured |
| | 1140 | 0.93 | Not measured |
| | 5700 | 0.97 | vNot measured |

When egg lecithin Lipoid E80 was replaced with phospholipon H 100, the cooled homogenate particle size was higher after the 10 passes than with Lipoid E80 equivalent (2.3 micrometers versus 0.3 micrometers, respectively). In addition after processing to form a cooled dispersion of small particles containing the drug, a further relative increase in particle size of cooled dispersion was detected. This can be attributed to aggregation of the primary particles. For both the Lipoid E80 formulation and the phospholipon H 100 formulation, aggregate sizes could be decrased over time with stirring.

Scanning electron microscopic (SEM) analysis of cooled dispersions prepared originally from fenofibrate and a phospholipid as a surface active substance in the admixture and by 10 volume passes revealed them to exist as single crystalline particles each about 1 micron in mean diameter. Cooled dispersions are comparable to microfluidized formulations of phospholipid and fenofibrate that can be prepared by microfluidization below the melting point of fenofibrate such as according to IDD-P™ technology developed by RTP Pharma Inc. as described in US Patent 5,091,187. However, to achieve a similar particle size reduction about a mean size without first melting the drug can require substantially more volume passes of microfluidization, for Example as many as 200 passes at ca. 18,000 psi. In addition, the particle size distribution is narrower when the hot melt method is used.

More than one surface active substance can be used to prepared formulations for use in this invention. At least one surface active substance is needed to prepare the initial admixture, and in one aspect can suffice in the preparation of subsequent heated suspensions. heated homogenates, cooled homogenates, cooled dispersions and dried particles prepared as described herein. In another aspect, addition of more than one surface active substance can be made to the admixture, the heated suspension, the heated homogenate, the cooled homogenate, and the cooled dispersion described herein. Such additions can be made at one individual step in the process or at more than one step in the process. For example, a second surface active agent can be added to the admixture or to the heated suspension, and additional amounts of the second surface active agent or a third surface active agent can be added to the cooled homogenate or to the cooled suspension or even to the dried small particles prepared as described herein.

The total concentration of one or of more than one surface active substance added to the formulations prepared as described herein can be in the range of 0.1 to 50%, preferably 0.2 to 20%, and more preferably 0.5 to 10%.

Bulking agents can be added to the admixture, to the heated homogenate, to the cooled homogenate, and to the cooled dispersion. Bulking agents can be added as solids, as mixtures, as solutions in aqueous carrier, and in combinations of solids and solutions. Bulking agents can be added at the beginning or end of the steps leading to the formation of a heated homogenate, cooled homogenate, and cooled dispersion, and they can be added at more than one stage during the process. The amount of total bulking agents that can be added ranges from about 0.1% to about 50%, preferably from 1% to about 25%, and more preferably from about 2% to about 20%. Bulking agents can be added as individual agents at these levels or in combination such that the total amount of bulking agent resides within these levels.

Addition of a variety of bulking agents at different steps in the process described herein does not produce a substantial increase the mean particle diameter of a cooled dispersion over a period of time such as over 24 hours. For example, when bulking agents sorbitol (5%) and sucrose (10%) were added to a 3% Lipoid E80 and 10% fenofibrate admixture and the formulation was processed for 10 passes to form a cooled homogenate and for 10 passes to form a cooled dispersion of small particles containing the drug, the particle size of the cooled dispersion (0.97 micrometers) was very similar in size to that of an analogous formulation composition (i.e., 0.91 micron) where the same bulking agents were added after the formation of the cooled dispersion.

Homogenization of the cooled homogenate containing the drug can be carried out in equipment suitable for that process. Useful equipment includes but is not limited to commercially available high pressure homogenization equipment such as APV Gaulin M15, Avestin Emulsiflex C5 or C50, MFIC Microfluidizer M110EH, and other microfluidizers and homogenizers. Homogenization can also be carried out using high shear and ultra high shear mechanical mixers and mills and propeller-containing mixers than can impart sufficient turbulence or energy transfer to the particles to form stable small particles. The apparatus is cooled to maintain the cooled homogenate and cooled dispersion at the second temperature range. Cooling can be done by use of a cooled air bath, a cooled fluid bath such as a water or ice/water bath, or a suitable heat exchanger that is cooled and maintained at or below the second temperature range that is below the melting point of the drug.

In a final step of the process to prepare microparticulate fenofibrate, the cooled dispersion can be dried to provide dry small particles containing the poorly soluble drug. Drying can be done using a number of commonly known methods, for example by spray drying, lyophilization, and evaporation. Preferably one or more than one bulking agent is present in the formulation undergoing drying.

When drying is done by spray drying the cooled dispersion is fed into the spray dryer as a liquid, preferably at a temperature in the second temperature range and preferably as a dispersion comprising one or more than one bulking agent. In one aspect, a preferred bulking agent can be selected from the group consisting of mannitol, sucrose, trehalose, sorbitol, and mixtures thereof. Additional pharmaceutically acceptable excipients such as Ac-Di-Sol and Cab-O-Sil can be added to the cooled dispersion prior to spray drying.

Spray drying of a cooled dispersion can be accomplished by methods known in the art using a commercially available spray drying apparatus such as a LabPlant SD05 Spray Dryer or using a larger scale spray drying apparatus. Preferably, dry air or dry oxygen free air or nitrogen or other non-oxidizing non-reactive dry gas is used in the spray drying. Moisture level in the isolated spray dried powder obtained as an initial product in the spray drying process is preferably below 3%, more preferably below 2%, and most preferably below 1%. Moisture level can be measured by a Karl Fisher method.

When drying is done by evaporation, the aqueous carrier of the cooled dispersion can be maintained as a liquid and water is removed under reduced pressure and with application of enough heat to keep at least some and preferably all of the aqueous carrier in the cooled dispersion that is drying in the liquid state until it is dried.

When drying is done by lyophilization, the aqueous carrier of the cooled dispersion is frozen and lyophilized under reduced pressure and application of heat to the frozen suspension to provide a dried lyophilizate comprising small particles containing poorly soluble drug. Freezing and lyophilization are preferably done in a conventional freeze dryer, for example, in a Virtis Corporation Unitop freeze dryer using conventional techniques. Lyophilization can be done on cooled dispersions added to trays or on cooled dispersions added to vials, for example in 2 mL or 10 mL vials. Bulking agents can be added to the formulation to facilitate reconstitution of the lyophilizate. In a preferred embodiment, bulking agents can be selected from the group consisting of mannitol, sucrose, sorbitol, trehalose, and combinations thereof. The amount of bulking agent present in the formulation can range from about 1% to about 50% or more. In a preferred embodiment, the amount of bulking agent can range from about 2% to about 20%, and in a more preferred embodiment, the amount of bulking agent can range from about 3% to about 15%.

In one aspect, the dried material can comprise phospholipid-stabilized particles in a bulking agent that is substantially amorphous. For example, the dried material can comprise phospholipid-stabilized particles of fenofibrate in substantially amorphous sucrose, in substantially amorphous mannitol, in substantially amorphous lactose, in a substantially amorphous mixture of sucrose and raffinose, in a substantially amorphous mixture of sucrose and sorbitol, in a substantially amorphous mixture of sucrose and raffinose and sorbitol. In a preferred embodiment, the dried material comprises phospholipid-stabilized particles of fenofibrate in a substantially amorphous bulking agent such as those listed previously, wherein the dried material contains from about 0.1% to about 3% of adsorbed water, more preferably 0.1% to about 2% of adsorbed water, and most preferably 0.1% to about 1% of adsorbed water. These values are below the absorption isotherm of an amorphous sugar containing microparticles of phospholipid stabilized fenofibrate. In one aspect, a dried formulation containing phospholipid-stabilized particles of fenofibrate in a substantially amorphous bulking agent will maintain its amorphous character if the amount of water present in the initially dried formulation is not increased for example by exposure to humidity that would lead to increased moisture content in the dried material and facilitate crystal growth. The rate of conversion of amorphous bulking agent to crystalline bulking agent can be enhanced by increasing the temperature and humidity to which the dried amorphous material is exposed. The rate of conversion of amorphous bulking agent to crystalline bulking agent can be reduced by decreasing the temperature and humidity to which the dried amorphous material is exposed.

In one theory, the rate of conversion of amorphous bulking agent to crystalline bulking agent can be related to the water absorption isotherm of the dried system that comprises the amorphous bulking agent, the phospholipid, and other excipients present in the formulation. If the amount of water or level of humidity to which the dried formulation is exposed is below the adsorption isotherm at a given temperature, the bulking agent will remain substantially amorphous and conversion to crystalline material will be relatively slow, preferably remaining substantially unchanged over 6 months, more preferably over 12 months, even more preferably over 18 months, and most preferably over 24 months; if the amount of water (humidity) to which the dried formulation is exposed is above the absorption isotherm at a given temperature, the amorphous material will tend to convert relatively rapidly to a crystalline material. The higher the level of humidity, the faster the conversion. The higher the temperature, the faster the conversion. A preferred keeping condition for an amorphous material described herein is thus about 4 °C to about 40 °C at a relative humidity level that is below the absorption isotherm of the amorphous material, more preferably from about 4 °C to about 30 °C at a relative humidity level that is below the absorption isotherm of the amorphous material, even more preferably from about 4 °C to about 25 °C at a relative humidity level that is below the absorption isotherm of the amorphous material, and most preferably from about 4 °C to about 20 °C at a relative humidity level that is below the absorption isotherm of the amorphous material.

In one embodiment, the dried amorphous material can be prepared by lyophilization. In this embodiment, the amorphous material can comprise a bulking agent in which the particles are suspended wherein the bulking agent is present as a glass. The glass can contain regions of crystalline bulking agent in addition to the particles of phospholipid stabilized microparticles. The amount of crystalline material can range from substantially zero to about 95% of the bulking agent, but is preferably less than 50%, more preferably less than 20%.

In another embodiment, the dried amorphous material can be prepared by spray drying. In this embodiment, the amorphous material can comprise a bulking agent in which the particles are suspended wherein the bulking agent is present as a bead. The bead can contain regions of crystalline bulking agent in addition to the particles of phospholipid stabilized microparticles. The amount of crystalline material can range from substantially zero to about 95% of the bulking agent, but is preferably less than 50°C, more preferably less than 20%.

In another aspect, the dried material can comprise phospholipid-stabilized particles in a bulking agent that is substantially crystalline. For example, the dried material can comprise phospholipid-stabilized particles of fenofibrate in substantially crystalline mannitol or substantially crystalline calcium phosphate.

In the case of fenofibrate as an example, in a final step of the process, the cooled dispersion can be dried by freezing the aqueous carrier in the dispersion and lyophilizating the frozen dispersion under reduced pressure and by application of heat to provide a dried lyophilizate comprising small particles containing fenofibrate. Optionally, the cooled suspension can be spray dried to provide a dried powder of particles containing fenofibrate. Alternatively, the water in the aqueous carrier of the cooled dispersion can be evaporated, for example under reduced pressure to provide dried small particles containing fenofibrate.

By small particles containing a poorly water soluble drug is meant particles in the range of 0.1 micron to 10 micrometers in average diameter containing a poorly water soluble drug, preferably in the range of 0.1 to 5 micrometers containing a poorly water soluble drug, and most preferably in the range of 0.1 to 2 micron containing a poorly water soluble drug.

By small particles containing fenofibrate is meant particles in the range of 0.1 micron to 10 micrometers in average diameter containing fenofibrate, preferably in the range of 0.1 to 5 micrometers containing fenofibrate, and most preferably in the range of 0.1 to 2 micron containing fenofibrate.

Addition of bulking agents such as sucrose, mannitol, trehalose, sorbitol and the like either to the admixture before processing or to the cooled dispersion just prior to drying provides particle size suspensions on reconstitution similar in size to those of the antecedent cooled dispersion. Drying can be done preferably by spray drying or by lyophilization. The presence of added water-insoluble excipients of sizes larger than the phospholipid stabilized fenofibrate microparticles present can be detected in particle size distribution measurements, but a size distribution of the microparticles of fenofibrate in the excipient-containing dried material will be substantially similar to that of the suspension of microparticles before drying.

Addition of bulking agent such as trehalose either to the admixture before processing, to the heated homogenate, to the cooled homogenate, or to the cooled dispersion just prior to drying provides particle size suspensions on reconstitution with an aqueous fluid that are similar in size to those of the antecedent cooled dispersion.

Samples of cooled homogenate can be dried for example by lyophilization with bulking agents and reconstituted in modified simulated gastric fluid (SGF) with gentle inversion immediately after lyophilization. The particle sizes of the dispersions on reconstitution are similar to, i.e., the same or slightly larger than, those of the antecedent cooled homogenates. Microscopically, the reconstituted suspensions can exist primarily as single crystalline particles together with occasional aggregates. For example, a cooled dispersion prepared from an admixture of 3% Lipoid E80 as the surface active substance, 10% fenofibrate, 10% sucrose, and 5% sorbitol as an antecedent cooled dispersion has an average particle size of 0.96 micrometers. On reconstitution of the corresponding lyophilizate, the average particle size of the reconstituted suspension is 1.57 micrometers. For the compositionally equivalent formulation where the bulking agents are added to the cooled dispersion, mean particle diameters before and after lyophilization are 0.91 and 1.38 micrometers, respectively.

Other bulking agents, for example glycerol at 2%, sucrose at 5%, also yield dried particles that reconstitute easily and provide suspensions of single crystalline particles.

The period of stability of the microparticles of small particles containing the drug in the cooled dispersion can extend from the stability period of the transiently stable particles of the cooled homogenate up to several months. Stability of more than a year is also contemplated.

Formulations prepared as described herein may be dried into powders which can be resuspended or filled into capsules or converted into granules or tablets with the addition of binders and other excipients known in the art of tablet making such as, for example, silica as a flow aid and magnesium stearate. A currently preferred capsule formulation for oral administration of phospholipid stabilized fenofibrate microparticles comprises fenofibrate (10 %w/w). as microparticles prepared by microfluidization in 10 mM phosphate buffer with phospholipid. Lipoid E80 (3 %w/w), sucrose (10 %w/w), and sorbitol (5, w/w). Other preferred formulations comprise fenofibrate (10%) as microparticles stabilized by phospholipid (e.g., Lipoid E80 at 0.5 to about 3%), and mannitol or sucrose (5% to 15%).

A suspension of microparticles such as one prepared by microfluidization of these ingredients is dried by lyophilization or by spray drying, optionally after being mixed with additional excipients such as Ac-Di-Sol, Cab-O-Sil, or other pharmaceutically acceptable excipients, to remove water and to form a solid which is blended with additional excipients and tableting agents known in the art such as colloidal silicon dioxide (about 1% w/w) and magnesium stearate (about 5 %w/w). This blend is then filled into capsules or compressed into tablets for oral delivery. The amount of fenofibrate per unit oral dosage form such as per capsule or tablet can range from about 50 mg to about 300 mg, but is preferably 50 mg, 67 mg, 100 mg, 134 mg, 150 mg, 160 mg, 200 mg, 213 mg, 250 mg, and 300 mg. Useful dosage levels for tablets and capsules include in the high end of the range milligram levels that are divisible by three such as 150 mg (giving related lower dosage levels of 100 mg and 50 mg), 159 mg (giving related lower dosage levels of 106 mg and 53 mg), 156 mg (giving related lower dosage levels of 104 mg and 52 mg), 153 mg (giving related lower dosage levels of 102 mg and 51 mg). Multiples of this type have the advantage of assisting a physician to titrate a patient to a therapeutically acceptable level starting with a low dose of the fibrate and changing the dose in well defined increments until a desired result is achieved, such as a lowering of levels of cholesterol, low density lipoproteins, and other species outlined in Table 1. Additional currently preferred dosage levels contain 50 mg, 67 mg, 100 mg, 134 mg, 150 mg, 160 mg, 200 mg and 213 mg of fenofibrate as microparticles stabilized with phospholipid.

Tablets and capsules and powders containing microparticles of fenofibrate for use in this invention can be packaged in bottles or blister packs or in other packaging for use by a human in need of treatment by fenofibrate. Preferably the packaging is sealed to substantially prevent the exposure of the tablets or capsules or powders to moisture. A blister packaging comprising aluminum foil sealed to exclude air containing moisture is a preferred packaging. A reclosable bottle or jar or other container comprising a lid or top or stopper as a closing means is preferably suitable if the closing means forms a seal with the remainder of the container that substantially prohibits the admission of air containing moisture to the contents which comprise the dried powders or tablets or capsule dosage forms for use in this invention. In a preferred container, a dessicant such as silica contained in a a moisture permeable package such as a closed sac or bag is present in the container proximal to the dosage forms to preferentially adsorb moisture.

Capsules and tablets and powders and granules for use in this invention for oral administration provide fenofibrate to a human patient in need of treatment by fenofibrate that is relatively independent of a food effect. Thus, a patient in a fasted state will receive at least 80 % of the dose of the drug active species that a patient in a fed state will receive by taking the same capsule or tablet or powder or granular dosage form (at the same level of drug per unit dosage form, i.e., at the same number of mg of drug per tablet or capsule given to the same patient when fasted as when fed). More preferably, a patient in a fasted state will receive at least 85 % of the dose of the drug active species that a patient in a fed state will receive by taking the same capsule or tablet or powder or granular dosage form. Even more preferably, a patient in a fasted state will receive at least 87 % of the dose of the drug active species that a patient in a fed state will receive by taking the same capsule or tablet or powder or granular dosage form. Even more preferably, a patient in a fasted state will receive at least 90 % of the dose of the drug active species that a patient in a fed state will receive by taking the same capsule or tablet or powder or granular dosage form. Even more preferably, a patient in a fasted state will receive at least 95 % of the dose of the drug active species that a patient in a fed state will receive by tasking the same capsule or tablet or powder or granular dosage form.

The tablets containing the fibrate dosage form for use in this invention can be prepared by compression of solid particles in a bulking agent such as a sugar as described herein. Optionally, the tablets can be coated with a pharmaceutically acceptable coating material such as pharmaceutically acceptable polymer for example carboxymethyl cellulose, sodium carboxymethyl cellulose, povidone, PVP, polyethylene, PEG, shellac, cellulose acetate, CAP, polyvinyl acetate phthalate, PVAP, hydroxypropyl methyl cellulose phthalate, HPMCP, polymers of methacrylic acid and its esters, Eudragit polymers, methyl cellulose, MC, ethyl cellulose, BC, hydroxyethyl cellulose, HEC, methylhydroxyethyl cellulose, MHEC, hydroxypropyl cellulose, HPC, hydroxypropylmethyl cellulose, HPMC, and combinations thereof and at revels well known in the art of tablet coating. The coatings can be applied in pharmaceutically acceptable form which is well known in the art such as suspension coating, fluid bed coating, spray coating, Escaravage coating which is coating method for individual tablets using a solution of coating materials applied with a brush, film coating, preferably from a water based solution and optionally from a water-solvent such as water-ethanol based solution, and dried to form a dried film-coating. The added weight to the tablet can be from about 0.1% to about 20%, preferably film 1% to about 5%. The solutions used to coat the tablet dosage form can of course optionally contain mixtures of ingredients such as sugars, pharmaceutically acceptable plastisizers, antioxidants, pH modifiers such as carboxylic acids or carboxylate salts, vitamin E, beta-carotene, and the like. The coating can be applied in a single layer or optionally in several layers with each layer being the same compositions or a different composition of ingredients.

Particles of drug provided for use in this invention have bioavailability comparable to or better than similar sized particles prepared by alternate methods. This is illustrated graphically in Figure 2 which compares the oral bioavailability of microparticles of fenofibrate prepared by microfluidization in the presence of a phospholipid stabilizing agent versus the oral bioavailability of micronized fenofibrate under fasting, low fat fed, and high fat fed conditions. In Figure 2A, the fenofibrate in microfluidized phospholipid-stabilized microparticles (bar 2) is nearly twice as bioavailable as that in a micronized formulation (bar 1) in the fasted state. In Figure 2B, the fenofibrate in microfluidized phospholipid-stabilized microparticles (bar 4) is more bioavailable than that in a micronized formulation (bar 3) in a low fat fed state. In Figure 2C, there is no significant difference in bioavailability between the fenofibrate in microfluidized phospholipid-stabilized microparticles (bar 6) and in a micronized formulation (bar 5). Bioavailability of fenofibrate increases by more than a factor of two when comparing bars 1, 3, and 5 that refer to a micronized formulation of fenofibrate. However, bioavailability of fenofibrate is approximately constant when comparing bars 2, 4, and 6 that refer to fenofibrate in a microfluidized phospholipid-stabilized microparticle formulation. The bioavailability of fenofibrate in formulations of microfluidized phospholipid-stabilized microparticles increases by less than 25% when comparing fasting and high fat fed conditions (e.g., bars 2 and 6), preferably increases by less than 20%, and more preferably increases by less than 15%. The clinical data used to produce bars 2 and 6 indicate an increase of 14% in the bioavailability of fenofibrate between fasted and high fat fed conditions, i.e., a factor of 1.14 between bioavailabilities represented by bar 2 (fasted) versus bar 6 (high fat fed). Blood levels of fenofibric acid were measured to obtain the data from which Figure 2 was generated.

The formulations for use in this invention comprising phospholipid stabilized microparticles of fenofibrate in the presence of a bulking agent formulated in a dosage form of fenofibrate (tablet, capsule, powder, dispersion of particles in a fluid, dispersion of particles in a nutrient such as a low fat food bar or other means of administering the particles) can be taken with or without food, especially when such food contains fat, to provide blood levels of fenofibrate active agent (i.e., fenofibric acid) that are substantially independent of the amount of food or fat in food (including fasting or zero fat, low fat, and high fat meals) taken proximal to the administration of the fenofibrate dosage form. This is a surprising result in view of the known food effect associated with other dosage forms of fenofibrates such as micronized fenofibrate and fenofibrate micronized in the presence of a solid surfactant such as sodium lauryl sulfate.

The invention is additionally illustrated in connection with the following examples, which are considered to be illustrative of the present invention. It should be understood, however, that the invention is not limited to the specific details of the Examples.

### Example 1.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15°C above the melting point of the drug, during continuous high shear mixing at 2,500 to 4,000 rpm. The heated suspension is then recirculatively homogenized for 10 batch volume cycles or passes using a Microfluidizer M110Y operated at 3,400 to 3,600 psig while maintained at 85 °C to 99 °C to form a heated homogenate containing the drug. After 10 passes, the heated homogenate is cooled by passage through a heat exchanger cooled by chilled water at 5 °C to 10 °C and the transiently stable cooled homogenate is further homogenized for 10 to 20 batch volume cycles. or passes using a Microfluidics M110 EH homogenizer operated at 18,000 psig (peak) while maintained at 4 °C to 13 °C. The resulting cooled dispersion comprising small particles containing fenofibrate of size less than 2.0 microns in diameter is then dried by freezing to about -40 °C and lyophilization under vacuum to produce dried small particles containing fenofibrate.

### Example 2.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y operated at 3,400 to 3,600 psig while maintained at 80 °C to form a heated homogenate containing the drug. After 10 passes, the heated homogenate is cooled by passage through a heat exchanger chilled with ice water, kept at 4 °C for 30 min, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles containing the drug are of a size less than 1.0 micron in diameter and are then dried by freezing and lyophilization under vacuum to produce dried small particles containing fenofibrate.

### Example 3.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer containing 240 parts of trehalose using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y homogenizer operated at 3,400 to 3,600 psig while maintained at 85 °C to 95 °C to form a heated homogenate containing the drug. After 10 passes, the heated homogenate is cooled by passage through a heat exchanger chilled with ice water, kept at 4 °C for 30 minutes in an ice/water bath, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles containing drug of size less than 1.0 micron in diameter is then dried by freezing in liquid nitrogen and lyophilization under vacuum to produce dried small particles containing fenofibrate.

### Example 4.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y homogenizer operated at 3,400 to 3,600 psig while maintained at 85 °C to form a heated homogenate containing drug. After 10 passes, the heated homogenate is cooled by passage through a heat exchanger chilled with ice water, kept at 4 °C for 30 min, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles containing the drug of size less than 1.0 micron in diameter is treated with a solution of 200 parts of sucrose plus 100 parts of sorbitol as bulking agents in additional aqueous carrier and is then dried by freezing in liquid nitrogen and lyophilization under vacuum to produce dried small particles containing fenofibrate.

### Example 5.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y homogenizer operated at 3,400 to 3,600 psig while maintained at 85 °C to form a heated homogenate containing drug. After 10 passes, the heated homogenate is cooled by passage through a heat exchanger chilled with ice water, kept at 4 °C for 30 min, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles containing the drug of size less than 1.0 micron in diameter is treated with a solution of bulking agents equivalent to 300 parts of sucrose plus 100 parts of sorbitol in additional aqueous carrier is then dried by freezing and lyophilization to produce dried small particles containing fenofibrate.

### Example 6.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y homogenizer operated at 3,400 to 3,600 psig while maintained at 85 °C to form a heated homogenate containing drug. After 10 passes, the heated homogenate is cooled by passage through a heat exchanger chilled with ice water, kept at 4 °C for 30 min, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles containing drug of size less than 1.0 micron in diameter is treated with 100 parts of sucrose plus 20 parts of glycerol as bulking agents, then dried to produce dried small particles containing fenofibrate.

### Example 7.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y homogenizer operated at 3,400 to 3,600 psig while maintained at 85 °C to form a heated homogenate containing drug. After 10 passes, the heated homogenate is cooled by passage through a heat exchanger chilled with ice water, kept at 4 °C for 30 min, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles containing drug of size less than 1.0 micron in diameter is treated with a cooled solution of 200 parts of trehalose plus 100 parts of PVP17 as bulking agents in additional aqueous carrier and then dried by freezing and lyophilization or by spray drying to produce dried small particles containing fenofibrate.

### Example 8.

A mixture of 60 parts of Lipoid E80 as the surface active substance and 200 parts of a poorly water soluble drug, fenofibrate, is homogeneously dispersed in 1440 parts of 10 mM pH 8.0 +/- 0.2 aqueous phosphate buffer containing 200 parts of sucrose and 100 parts of sorbitol using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y homogenizer operated at 3,400 to 3,600 psig while maintained at 80 °C to form a heated homogenate containing drug. After 10 passes, the heated homogenate is cooled by passage through a heat exchanger chilled with ice water, kept at 4 °C for 30 min, and the transiently stable cooled homogenate is further homogenized for 10 to 20 batch volume cycles or passes using a Microfluidics M110 EH homogenizer operated at 18,000 psig (peak) while maintained between 4 °C and 15 °C. The resulting cooled dispersion comprising small particles of size less than 1.0 micrometers in diameter is then dried to produce dried small particles containing fenofibrate.

### Example 9.

An admixture of a formulation comprising 60 parts of a hydrogenated soybean phosphatidylcholine (i.e., phospholipon 100H) as a surface active substance and 200 parts of a poorly water soluble drug, fenofibrate, in 1400 parts of aqueous carrier (10 mM phosphate buffer at pH 8) is heated to 85 °C and homogenized for 10 volume passes to form a heated homogenate containing drug containing the drug, cooled to room temperature according to method 1 to form a transiently stable cooled homogenate containing the drug, and then sonicated for 1 minute using a 550 Sonic Dismembrator Probe Sonicator from Fisher Scientific (10 s pulses at power level 5) to form a cooled dispersion. The mean particle diameter of the sonicated material (cooled dispersion) is only slightly larger than that of the heated homogenate material, both being between 2-4 micrometers. Microscopically, the heated homogenate particles are non-crystalline while the cooled dispersion particles are crystalline. Importantly, while stirring induces significant particle growth in the cooled homogenate, stirring does not induce significant particle growth in the cooled dispersion. The cooled dispersion thus produced is more robust toward particle growth than the cooled homogenate.

### Example 10.

A mixture of 60 parts of a phospholipid as a surface active substance and 200 parts of a poorly water soluble drug is homogeneously dispersed in 1440 parts of 10 mM pH 8.0 +/- 0.2 aqueous phosphate buffer using a ProScientific 400 high shear mixer at 2,000 to 3,600 rpm at ambient temperature for 30 minutes, and then heated above the melting point of the drug during continuous high shear mixing at 2,500 to 4,000 rpm. The heated suspension is then recirculatively homogenized for 10 batch volume cycles or passes using a Microfluidizer M110Y operated at 3,400 to 3,600 psig while maintained above the melting point of the drug to form a heated homogenate containing drug. After 10 passes, the heated homogenate is cooled by passage through a heat exchanger chilled with ice water, and the transiently stable cooled homogenate is further homogenized for 10 to 20 batch volume cycles or passes using a Microfluidics M110 EH homogenizer operated at 18,000 psig (peak) while maintained at 4 °C to 15 °C. The resulting cooled dispersion comprising particles containing the poorly water soluble drug is then dried by freezing and lyophilization to produce dried small particles containing the poorly water soluble drug.

### Example 11

Cooled dispersions prepared according to examples 1 to 9 are placed into 10 ml vials and individually frozen and lyophilized to provide dried small particles containing fenofibrate.

### Example 12

Cooled dispersions prepared according to examples 1 to 9 are individually spray dried to provide dried small particles containing fenofibrate.

### Example 13

A cooled dispersion prepared according to example 10 using fenofibrate is placed in 10 ml vials, frozen and lyophilized to provide dried small particles containing fenofibrate.

### Example 14

A cooled dispersion prepared according to example 10 using fenofibrate is spray dried to provide dried small particles containing fenofibrate.

### Example 15

A mixture of 225 parts of Lipoid E80 as the surface active substance, 750 parts of fenofibrate, 375 parts of sorbitol, and 750 parts of sucrose is homogeneously dispersed in 6000 parts of 10 mM pH 8.0 +/- 0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension is then recirculatively homogenized for 10 batch volume cycles or passes using a Microfluidizer M110Y operated at 3,400 to 3,600 psig while maintained at 85 °C to 99 °C to form a heated homogenate containing the drug. After 10 passes, the heated homogenate is cooled by passage through a heat exchanger cooled by chilled water at 5 °C to 10 °C and the transiently stable cooled homogenate is further homogenized for 10 to 20 batch volume cycles or passes using a Microfluidics M110 EH homogenizer operated at 18,000 psig (peak) while maintained at 4 °C to 13 °C. The resulting cooled dispersion comprising small particles containing fenofibrate of size less than 1.0 micron in diameter is then dried by freezing to about -40 °C and lyophilization under vacuum to produce dried small particles containing fenofibrate.

### Example 16.

The dried small particles containing fenofibrate prepared in Example 15 are blended with 2 % Cabosil, 5 % sucrose, and 0.25% magnesium stearate. After thorough blending, the mixture is compressed, optionally with an intermediate formation of compressed slugs of the composition which are milled, optionally sieved to a uniform particle size range, and then recompressed into tablets for oral dosing. The tablets are prepared at the following dosage levels of fenofibrate and are sized according to volumes encountered.
50 mg
51 mg
52 mg
53 mg
54 mg
67 mg
100 mg
102 mg
104 mg
106 mg
134 mg
150 mg
153 mg
156 mg
159 mg
160 mg
200 mg
213 mg
250 mg
300 mg

### Example 17.

Gelatin capsules are filled with the dried small particles containing fenofibrate prepared in Example 15 and sealed to provide capsules for oral dosing. The capsules are filled at the following dosage levels of fenofibrate and are sized according to volumes encountered.
50 mg
51 mg
52 mg
53 mg
54 mg
67 mg
100 mg
102 mg
104 mg
106 mg
134 mg
150 mg
153 mg
156 mg
159 mg
160 mg
200 mg
213 mg
250 mg
300 mg

### Example 18.

Oral bioavailability of a microfluidized phospholipid-stabilized microparticle formulation of fenofibrate in human subjects.

An oral capsule dosage form of a formulation of microfluidized Phospholipon 100H-stabilized fenofibrate microparticles (67 mg dose of fenofibrate) prepared with Tween 80 and mannitol was administered to human volunteers. The study consisted of oral administration of capsules containing a formulation of microfluidized Phospholipon 100H-stabilized fenofibrate microparticles to eight human volunteers in a single dose crossover design, using a commercially marketed formulation of micronized fenofibrate as a reference. The dose administered was 67 mg. Blood samples were collected before and after each administration at various time points over 120 hours. The drug concentration in blood samples was determined by high-pressure liquid chromatography by monitoring for the level of the metabolite, fenofibric acid. The pharmacokinetic results are presented in Table 5. The ratio of the least-squares means (In-transformed data) was 1.49 ± 0.24, and demonstrate the superior bioavailability of fenofibrate in the microfluidized phospholipid-stabilized fenofibrate microparticle formulation over the commercially available product.

| Table 5. Cₘₐₓ and AUC_{0-inf} for Fenofibric Acid | | |
|---|---|---|
| | Cₘₐₓ (ng.ml⁻¹) | AUC_{0-∞} (ng.ml⁻¹.h) |
| Microfluidized phospholipid- stabilized fenofibrate microparticle | 2528 | 57236 |
| formulation (67 mg) | | |
| Commercially available micronized | 1372 | 38629 |
| fenofibrate (67 mg) product | | |
| Dunnett's t-test (log-transformed | p<0.05 | p<0.05 |
| data) | | |

### Example 19.

Elimination of the food effect associated with marketed formulations of fenofibrate using a microfluidized phospholipid-stabilized microparticle formulation of fenofibrate in human subjects.

The oral bioavailability of a capsule dosage form of a microfluidized phospholipid-stabilized microparticle formulation of fenofibrate comprising Phospholipon 100H-stabilized fenofibrate microparticles prepared by microfluidization, Tween 80, and mannitol was tested and compared with the marketed micronized formulation of fenofibrate in fasting and fed states in a single dose pharmacokinetic study. The study consisted of the oral administration of capsules of the test formulations to 8 human subjects in a single dose, crossover design with four treatment periods. Both drug formulations were administered as 67 mg capsules. Blood samples were collected before and after each administration at various time points over 120 hours. The drug concentration in blood samples was determined by high-pressure liquid chromatography by monitoring for the level of the metabolite, fenofibric acid. The bioavailability (AUC_{0-∞}) under the different conditions is presented in Table 6. The food effect is represented by the ratio of the AUC_{0-∞} under fed and fasted conditions. The results demonstrate a significant (p<0.05) food effect with the marketed micronized fenofibrate product (+73%), while the food effect with the microfluidized phospholipid stabilized microparticle fenofibrate was only 13% (NS), demonstrating the virtual elimination of the dependence on food for optimal bioavailability.

| Table 6. AUC_{0-∞}. for fenofibric acid under fasted and fed conditions | | |
|---|---|---|
| AUC_{0-∞} (ng.ml⁻¹.h) | Microfluidized phospholipid stabilized microparticle fenofibrate (67 mg) | Marketed micronized fenofibrate product (67 mg) |
| Fasting state | 57236 | 38629 |
| Fed state | 64585 | 66969 |
| Fᵣₑₗ (fed / fasted) | 1.13 | 1.73 |
| Dunnett's t-test (In-transformed data) | NS | p<0.05 |

### Example 20.

Demonstration of the absence of food effect with a microfluidized phospholipid-stabilized microparticle formulation of fenofibrate (IDD-P™ fenofibrate) in human subjects

An IDD-P™ fenofibrate formulation prepared by a hot melt microfluidization process described herein under GMP conditions according to the method of Example 15 was dried by lyophilization and formulated into tablets containing 160 mg of fenofibrate. In the formulation, the IDD-P™ fenofibrate was in the form of microfluidized microparticles stabilized by phospholipid Lipoid E80 and was prepared by microfluidization in the presence of sucrose and sorbitol. The oral bioavailability of the tableted IDD-P™ fenofibrate formulation was tested in the fasting and fed states in a single dose pharmacokinetic study. The study consisted of the administration of a single IDD-P™ fenofibrate tablet containing 160 mg of fenofibrate in 8 human subjects using a crossover design with randomized sequences. The fed condition was obtained with a high fat meal containing 1000 Kcal and 50 g fat. The blood samples were collected before and after each administration at various time points over 96 hours. The drug concentration in blood samples was determined by high-pressure liquid chromatography by monitoring for the level of the metabolite, fenofibric acid. The bioavailability of the drug from a dosage form such as an orally administered composition of the drug is given by the accumulated amount of drug versus time detected in a patient, and is calculated as the area under the curve of a plot of fenofibric acid concentrations detected in blood versus time. The bioavailability *(AUC*₀*_{-∞})* data obtained under the fed and fasted conditions are presented in Table 7. The food effect is represented by the ratio of the *AU*C*_{0-∞}* under fed and fasted conditions. The ratio of 95% (fasted/fed) demonstrates the essential absence of food effect on the bioavailability of IDD-P™ fenofibrate. The ratio of the *AUC*₀*_{-∞}* under fasted/fed conditions is 1.07. Thus the bioavailability of microfluidized phospholipid stabilized microparticles of fenofibrate increases by less than 8% between fasted and fed conditions in this example.

| Table 7. AUC_{0-∞}. for fenofibric acid under fasted and fed conditions | |
|---|---|
| | AUC_{0-∞} (ng.ml⁻¹.h) |
| Fasting state | 126282 |
| Fed state | 135201 |
| Fᵣₑₗ (fasted/fed)⁽¹⁾ | 0.95 |
| ⁽¹⁾ Ratio of the least-squares means using In-transformed data | |

### Example 21.

The following formulations were prepared according to the method of example 10 leading to a suspension before drying:
21-1) 10% fenofibrate, 3% Lipoid E80, 10% sucrose;
21-2) 10% fenofibrate, 3% Lipoid E80, 10% sucrose, 5% sorbitol;
21-3) 10% fenofibrate, 3% Lipoid E80, 10% sucrose, 1% sorbitol;
21-4) 9% fenofibrate, 2.7% Lipoid E80, 19% sucrose, 4.5% sorbitol.

The formulations were spray dried in a commercially available spray dryer consisting of a chamber with inside diameter of 1.22 meters and a cylindrical height of 1.14 meters with a 60° conical bottom. Electrically heated air was used as the process gas admitted via a ceiling disperser. Each spray dried formulation was isolated initially as a dried powder that could be handled in a dry atmosphere without caking. A sample of spray dried powder prepared from formulation 21-2 that had an initial volume weighted average particle size of 1.7 microns in suspension before spray drying was reconstituted with mild sonication in simulated gastric fluid comprised of 2g NaCl and 7 ml of conc. HCl per liter and found to have an average particle size of 1.9 microns.

### Example 22.

A mixture of Lipoid E80 and fenofibrate was homogeneously dispersed in 10 mM pH 8.0 +/-0.2 aqueous phosphate buffer using a ProScientific 400 high shear mixer at 2,000 to 3,600 rpm at ambient temperature for 30 minutes, and then heated to 95 °C, 15 °C above the melting point of the drug, during continuous high shear mixing at 2,500 to 4,000 rpm. The heated suspension was then batchwise homogenized in 3 to 10 batch volume cycles using a Microfluidizer M110Y operated at 3,400 to 3,600 psig while maintained at 85 °C to 99 °C to form a heated homogenate containing the drug. The heated homogenate was cooled by passage through a heat exchanger cooled by chilled water at 5 °C to 10 °C and the transiently stable cooled homogenate was further homogenized for 10 to 20 batch volume cycles using a Microfluidics M110 EH homogenizer operated at 18,000 psig (peak) while maintained below 13 °C. The resulting cooled dispersion comprising small particles containing fenofibrate stabilized with phospholipid was then treated with bulking agents and excipients, mixed at ambient temperature, and then dried by spray drying. The following compositions (in wt %) were prepared by this method as powders having volume weighted diameter after reconstitution with mild sonication of 1 to 2 microns with smallest mode (vol. wt) unsonicated as 1.5 microns. The powders produced were easily flowing, easily transferable by pouring, and exhibited no sticking. Water content in these powders was found to be less than 2.5%, and in some cases such as 22-e, about 1%.

| Suspension | Fenofibrate | Lipoid E80 | Sucrose | Mannitol | Ac-Di-Sol | Cab-O-Sil |
|---|---|---|---|---|---|---|
| No. | | | | | | (colloidal silica) |
| 22-a | 10.0 | 0.5 | 17.5 | | | |
| 22-b | 10.0 | 0.5 | 17.5 | | 1.8 | |
| 22-c | 10.0 | 0.5 | 17.5 | | | 0.5 |
| 22-d | 10.0 | 0.5 | 7 | | 3 | 0.5 |
| 22-e | 10.0 | 0.5 | | 7 | 3 | 0.5 |
| 22-f | 10.0 | 0.5 | 17.5 | | 1.8 | 0.5 |

Spray dried powders (100 parts) were blended with excipients Avicel-PH102 (18.5 parts), Ac-Di-Sol (3.95 parts), Cab-O-Sil (0.62 parts), and magnesium stearate (0.25 parts), processed into 1 mm granules or slugs by preliminary compression of the blend followed by crushing and seiving (USP Standard #14 sieve), blended with additional magnesium sterarate, and then compressed into tablet dosage forms. Hardness of the tablets produced in different batches ranged from 2 to 9 KPa either in an automatic tableting machine or by manual compression using a CMS-15 tablet press (Cadmach Machinaries). Disintegration times of these tablets were in the range of 3 to 10 minutes.

### Example 23.

A two-treatment, two-period, two-sequence crossover clinical study was performed to evaluate the relative bioavailability of fenofibric acid in blood in 24 healthy volunteers after single dose oral administration of a tablet formulation of this invention comprising phospholipid stabilized microparticles of fenofibrate. The fenofibrate tablet dosage form consisted of 160 mg of fenofibrate and was derived from a dried lyophilized powder of this invention that contained between 0.1% and 3% moisture, and that was obtained from a suspension of microparticles consisting of 10% fenofibrate, 3% Lipoid E80, 10% sucrose, and 5% sorbitol, and that was further blended with sucrose at 5% by weight of the powder plus magnesium stearate at 0.2% plus colloidal silica at 0.2%. The bioavailability of fenofibric acid from the formulation of this invention was compared relative to that of commercially available micronized fenofibrate (Tricor^{®}) in a 200 mg capsule. Each dosage form was taken orally within 5 minutes after a low-fat test meal. The study was divided into 2 study periods, study period 1 and study period 2. At each period a single fenofibrate dose was administered to the subjects. There was a washout period of 10 days between the 2 administrations. Plasma samples were collected before each administration and during the 96 hours following each administration. Assay of fenofibric acid was performed with a validated analytical method (HPLC-UV) on the plasma samples. Relevant pharmacokinetic parameters were determined to evaluate the bioavailability of fenofibric acid after administration of each formulation, and the test formulation was compared to the reference formulation. The following results demonstrate bioequivalence between the formulation of this invention and the commercially available micronized fenofibrate (Tricor^{®}) under low fat fed conditions.

| Parameters (N =24) | 160 mg fenofibrate formulation of this invention fed with a low fat meal | | | 200 mg Tricor^{®} fed with a low fat meal | | |
|---|---|---|---|---|---|---|
| | Mean | +/- SD | CV (%) | Mean | +/- SD | CV(%) |
| AUC₀₋ₜ = experimental area under the curve calculated according to the linear trapezoidal rule (ng.h/mL) | 137587.71 | 48203.28 | 35.03 | 149272.07 | 58621.21 | 39.27 |
| AUC_{0-∞} = area under the curve extrapolated to the infinite (ng.h/mL) | 140067.57 | 49380.22 | 35.25 | 152599.13 | 60529.39 | 39.67 |
| *C*ₘₐₓ= maximal plasma concentration (ng/mL) | 11204.05 | 2507.73 | 22.38 | 10401.84 | 3039.54 | 29.22 |
| % extrapolated | 1.76 | 1.13 | 63.91 | 2.12 | 1.22 | 57.83 |
| *t*ₘₐₓ = time to reach the maximal plasma concentration (hours, h) | 3.21 | 1.10 | 34.36 | 4.75 | 0.90 | 18.88 |
| *k*ₑₗ = elimination rate constant (h⁻¹) | 0.0507 | 0.0220 | 43.51 | 0.0449 | 0.0177 | 39.37 |
| t_{1/2 el} = half-life of elimination (h) | 15.72 | 5.47 | 34.76 | 17.77 | 6.51 | 36.63 |
| *F*_{r*e*l} = relative bioavailability (%) | 94.05 | 12.36 | 13.14 | 100.00 | 0.00 | - |

| | AUC₀₋ₜ | | AUC_{0-∞} | | Cₘₐₓ | |
|---|---|---|---|---|---|---|
| Ratio of LS Means calculated using least squares means (In- transformed data) | 94.09% | | 93.69% | | 110.73% | |
| Ratio of Arithmetic Means calculated using arithmetic means (untransformed data) | 92.17% | | 91.79% | | 107.71% | |
| 90% Geometric Confidence Interval using In-transformed data | 89.15% to 99.31% | | 89.09% to 98.53% | | 101.84% to 120.39% | |
| Intra-Subject CV | 10.27% | | 9.58% | | 15.98% | |

Figure 3A is a graph of fenofibric acid mean plasma concentration (in ng/ml) versus time (in hours) found after oral administration of a 160 mg fenofibrate-containing tablet prepared according to this invention compared to that of a commercially available 200 mg Tricor^{®} capsule each taken proximal to ingestion of a low fat meal (n=24). Data were derived from the present study described in this Example and demonstrate statistical bioequivalence between the two dosage forms under low fat fed conditions.

Figure 3B is a graph of fenofibric acid Ln mean plasma concentration (in ng/ml) versus time (in hours) found after oral administration of a 160 mg fenofibrate-containing tablet prepared according to this invention compared to that of a commercially available 200 mg Tricor^{®} capsule each taken proximal to ingestion of a low fat meal (n=24). Data were derived from the present study described in this Example and demonstrate statistical bioequivalence between the two dosage forms under low fat fed conditions.

## Claims

1. The use of a composition comprising fenofibrate microparticles and a phospholipid surface active substance to reduce variance in the bioavailability of fenofibrate in a mammal in a fasting state versus a mammal in a fed state, in the manufacture of a medicament for the treatment of dislipidemia or dislipoproteinemia by oral administration of said medicament to the mammal such that the bioavailability of fenofibrate in a fasting mammal is increased by less than 25 % compared with the bioavailability of fenofibrate in a fed mammal and/or such that the bioavailability of fenofibrate is increased by a factor of 1.14 in a fasted mammal versus a fed mammal.

2. The use according to claim 1, wherein the dislipidemia comprises hypercholesterolemia, hyperlipidemia, hypertriglyceridemia, or a combination thereof.

3. The use according to claim 1 or claim 2, wherein the orally administrated medicament is selected from the group consisting of a tablet, a film-coated tablet, a moisture resistant tablet, a tablet coated with a pharmaceutically acceptable polymer and a capsule.

4. The use according to any one of claims 1 to 3, wherein the phospholipid surface active substance is a single phospholipid or a mixture of phospholipids selected from the group consisting of egg-derived phospholipid, Lipoid E80, Lipoid EPC. Lipoid SPC, dimyristoyl phosphatidyl glycerol (DMPG), Phospholipon 100H, a hydrogenated soybean phosphatidylcholine, Phospholipon 90H and Lipoid SPC-3.

5. The use according to any one of claims I to 4, wherein the phospholipid surface active substance is present in the composition in an amount of 0.1 to 50%.

6. The use according to claim 5, wherein the phospholipid surface active substance is present in the composition in an amount of 0.2 to 20 %.

7. The use according to claim 6, wherein the phospholipid surface active substance is present in the composition in an amount of 0.5 to 10 %.

8. The use according to claim 4, wherein the phospholipid surface active substance is Lipoid E80.

9. The use according to claim 8, wherein the Lipoid E80 phospholipid surface active substance is present in the composition in an amount from 0.5% to 15%.

10. The use according to claim 8 or claim 9, wherein the Lipoid E80 phospholipid surface active substance is present in the composition in an amount from 0,5% to 10%.

11. The use according to any one of claims 8 to 10, wherein the Lipoid E80 phospholipid surface active substance is present in the composition in an amount from 0.5% to 5%.

12. The use according to any one of claims 1 to 11, wherein the composition further comprises an aqueous carrier.

13. The use according to claim 12, wherein the aqueous carrier is selected from water, sterile water, buffered water, or phosphate buffered water.

14. The use according to claim 12 or 13, wherein the concentration of fenofibrate in the aqueous carrier is from 0.1% w/w to 90% w/w.

15. The use according to claim 14, wherein the concentration of fenofibrate in the aqueous carrier is from 0.5% w/w to 50% w/w.

16. The use according to claim 15, wherein the concentration of fenofibrate in the aqueous carrier is from 1% w/w to 20% w/w.

17. The use according to any one of claims 1 to 16, wherein the microparticles have an average particle size of less than 10 microns.

18. The use according to any one of claims 1 to 17, wherein the microparticles have an average particle size of less than 0.5 microns.

## Patentansprüche

1. Verwendung einer Zusammensetzung, die Fenofibrat-Mikroteilchen und eine oberflächenaktive Phospholipid-Substanz umfasst, zur Verminderung der Veränderung der Bioverfügbarkeit von Fenofibrat in einem Säuger in einem Nahrungskarenzzustand gegenüber einem Säuger in einem mit Nahrung versorgten Zustand, bei der Herstellung eines Medikaments zur Behandlung von Dyslipidämie oder Dyslipoproteinämie durch orale Verabreichung des Medikaments an den Säuger, so dass die Bioverfügbarkeit von Fenofibrat in einem fastenden Säuger um weniger als 25 % verglichen mit der Bioverfügbarkeit von Fenofibrat in einem Säuger, der Nahrung erhält, erhöht wird, und/oder die Bioverfügbarkeit von Fenofibrat um einen Faktor von 1,14 in einem fastenden Säuger gegenüber einem Säuger, der Nahrung erhält, erhöht wird.

2. Verwendung nach Anspruch 1, bei der die Dyslipidämie Hyperchol-esterinämie, Hyperlipidämie, Hypertriglyceridämie oder eine Kombination davon umfasst.

3. Verwendung nach Anspruch 1 oder Anspruch 2, bei der das oral verabreichte Medikament aus der Gruppe, bestehend aus einer Tablette, einer filmbeschichteten Tablette, einer feuchtigkeitsbeständigen Tablette, einer mit einem pharmazeutisch verträglichen Polymer beschichteten Tablette und einer Kapsel, ausgewählt ist.

4. Verwendung nach einem der Ansprüche 1 bis 3, bei der die oberflächenaktive Phospholipidsubstanz ein einzelnes Phospholipid oder ein Gemisch von Phospholipiden ist, ausgewählt aus der Gruppe, bestehend aus von Eiern stammendem Phospholipid, Lipoid E80, Lipoid EPC, Lipoid SPC, Dimyristoylphosphatidylglycerin (DMPG), Phospholipon 100H, einem hydrierten Sojabohnenphosphatidylcholin, Phospholipon 90H und Lipoid SPC-3.

5. Verwendung nach einem der Ansprüche 1 bis 4, bei der die oberflächenaktive Phospholipidsubstanz in der Zusammensetzung in einer Menge von 0,1 bis 50 % vorliegt.

6. Verwendung nach Anspruch 5, bei der die oberflächenaktive Phospholipidsubstanz in der Zusammensetzung in einer Menge von 0,2 bis 20 % vorliegt.

7. Verwendung nach Anspruch 6, bei der die oberflächenaktive Phospholipidsubstanz in der Zusammensetzung in einer Menge von 0,5 bis 10 % vorliegt.

8. Verwendung nach Anspruch 4, bei der die oberflächenaktive Phospholipidsubstanz Lipoid E80 ist.

9. Verwendung nach Anspruch 8, bei der die oberflächenaktive Phospholipidsubstanz Lipoid E80 in der Zusammensetzung in einer Menge von 0,5 % bis 15 % vorliegt.

10. Verwendung nach Anspruch 8 oder Anspruch 9, bei der die oberflächenaktive Phospholipidsubstanz Lipoid E80 in der Zusammensetzung in einer Menge von 0,5 % bis 10 % vorliegt.

11. Verwendung nach einem der Ansprüche 8 bis 10, bei der die oberflächenaktive Phospholipidsubstanz Lipoid E80 in der Zusammensetzung in einer Menge von 0,5 % bis 5 % vorliegt.

12. Verwendung nach einem der Ansprüche 1 bis 11, bei der die Zusammensetzung ferner einen wässrigen Träger umfasst.

13. Verwendung nach Anspruch 12, bei welcher der wässrige Träger aus Wasser, sterilem Wasser, gepuffertem Wasser oder Phosphat-gepuffertem Wasser ausgewählt ist.

14. Verwendung nach Anspruch 12 oder 13, bei der die Konzentration von Fenofibrat in dem wässrigen Träger von 0,1 % Gew./Gew. bis 90 % Gew./Gew. beträgt.

15. Verwendung nach Anspruch 14, bei der die Konzentration von Fenofibrat in dem wässrigen Träger von 0,5 % Gew./Gew. bis 50 % Gew./Gew. beträgt.

16. Verwendung nach Anspruch 15, bei der die Konzentration von Fenofibrat in dem wässrigen Träger von 1 % Gew./Gew. bis 20 % Gew./Gew. beträgt.

17. Verwendung nach einem der Ansprüche 1 bis 16, bei der die Mikroteilchen eine durchschnittliche Teilchengröße von weniger als 10 Mikrometer aufweisen.

18. Verwendung nach einem der Ansprüche 1 bis 17, bei der die Mikroteilchen eine durchschnittliche Teilchengröße von weniger als 0,5 Mikrometer aufweisen.

## Revendications

1. Utilisation d'une composition comprenant des microparticules de fenofibrate et une substance tensioactive de type phospholipide pour réduire la variance de biodisponibilité du fénofibrate entre un mammifère à jeun et un mammifère ayant mangé, dans la fabrication d'un médicament pour le traitement de la dyslipidémie ou de la dyslipoprotéinémie par administration par voie orale dudit médicament au mammifère de façon que la biodisponibilité du fénofibrate chez un mammifère à jeun soit augmentée de moins de 25 % par rapport à la biodisponibilité du fénofibrate chez un mammifère ayant mangé, et/ou de façon que la biodisponibilité du fenofibrate soit augmentée d'un facteur de 1,14 chez un mammifère à jeun par rapport à un mammifère ayant mangé.

2. Utilisation selon la revendication 1, dans laquelle la dyslipidémie comprend l'hypercholestérolémie, l'hyperlipidémie, l'hypertriglycéridémie, ou une de leurs combinaisons.

3. Utilisation selon la revendication 1 ou la revendication 2, dans laquelle le médicament administré par voie orale est choisi dans l'ensemble constitué par un comprimé, un comprimé pelliculé, un comprimé résistant à l'humidité, un comprimé enrobé d'un polymère pharmaceutiquement acceptable, et une capsule.

4. Utilisation selon l'une quelconque des revendications 1 à 3, dans laquelle la substance tensioactive de type phospholipide est un phospholipide unique ou un mélange de phospholipides choisis dans l'ensemble constitué par le phospholipide d'oeuf, le Lipoid E80, le Lipoid EPC, le Lipoid SPC, le dimyristoyl-phosphatidylglycérol (DMPG), le Phospholipon 100H, une phosphatidylcholine de soja hydrogénée, le Phospholipon 90H et le Lipoid SPC-3.

5. Utilisation selon l'une quelconque des revendications 1 à 4, dans laquelle la substance tensioactive de type phospholipide est présente dans la composition en une quantité de 0,1 à 50 %.

6. Utilisation selon la revendication 5, dans laquelle la substance tensioactive de type phospholipide est présente dans la composition en une quantité de 0,2 à 20 %.

7. Utilisation selon la revendication 6, dans laquelle la substance tensioactive de type phospholipide est présente dans la composition en une quantité de 0,5 à 10 %.

8. Utilisation selon la revendication 4, dans laquelle la substance tensioactive de type phospholipide est le Lipoid E80.

9. Utilisation selon la revendication 8, dans laquelle la substance tensioactive de type phospholipide Lipoid E80 est présente dans la composition en une quantité de 0,5% à 15 %.

10. Utilisation selon la revendication 8 ou la revendication 9, dans laquelle la substance tensioactive de type phospholipide Lipoid E80 est présente dans la composition en une quantité de 0,5 % à 10 %.

11. Utilisation selon l'une quelconque des revendications 8 à 10, dans laquelle la substance tensioactive de type phospholipide Lipoid E80 est présente dans la composition en une quantité de 0,5 % à 5 %.

12. Utilisation selon l'une quelconque des revendications 1 à 11, dans laquelle la composition comprend en outre un véhicule aqueux.

13. Utilisation selon la revendication 12, dans laquelle le véhicule aqueux est choisi parmi l'eau, l'eau stérile, l'eau tamponnée, et l'eau tamponnée au phosphate.

14. Utilisation selon la revendication 12 ou 13, dans laquelle la concentration de fénofibrate dans le véhicule aqueux est de 0,1 % plu à 90 % p/p.

15. Utilisation selon la revendication 14, dans laquelle la concentration de fénofibrate dans le véhicule aqueux est de 0,5 % p/p à 50 % p/p.

16. Utilisation selon la revendication 15, dans laquelle la concentration de fénofibrate dans le véhicule aqueux est de 1 % p/p à 20 % p/p.

17. Utilisation selon l'une quelconque des revendications 1 à 16, dans laquelle les microparticules ont une granulométrie moyenne inférieure à 10 micromètres.

18. Utilisation selon l'une quelconque des revendications 1 à 17, dans laquelle les microparticules ont une granulométrie moyenne inférieure à 0,5 micromètre.
